# EUROPEAN PATENT APPLICATION

(11) **EP 3 608 669 A1**
(43) Date of publication of application: **12.02.2020**
(21) Application number: 17904947.3
(22) Date of filing: 07.04.2017
(51) Int. Cl.: G01N 33/543

(54) **FLUORESCENT PREMIX PARTICLES, FLUORESCENT STAIN CONTAINING SAME, AND FLUORESCENT STAINING METHOD IN WHICH THESE ARE USED**

(71) Applicant: Konica Minolta, Inc., Tokyo 100-7015 (JP)
(72) Inventor: ISODA, Takeshi, Tokyo 100-7015 (JP); TOMIOKA, Daisuke, Tokyo 100-7015 (JP)
(74) Representative: MFG Patentanwälte Meyer-Wildhagen Meggle-Freund Gerhard PartG mbB
(86) International application number: PCT/JP2017/014569
(87) International publication number: WO 2018/185943

(57) **Abstract**

The present invention relates to fluorescent premix particles, a fluorescent stain containing the same, and a fluorescent staining method in which these are used, and the fluorescent premix particles are particles including: phosphor integrated particles that are modified with a first reactive substance; and at least one target protein-binding substance that is modified with a second reactive substance and is selected from the group consisting of an antibody and an aptamer, wherein the phosphor integrated particles and the at least one target protein-binding substance are linked by interaction between the first reactive substance and the second reactive substance.

## Description

### Technical Field

The present invention relates to fluorescent premix particles, a fluorescent stain containing the same, and a fluorescent staining method in which these are used.

### Background Art

Pathological diagnosis is a method of determining various events, for example, whether a patient suffers from a specific disease or whether a specific therapeutic agent is effective, by observing the morphology of cells or tissues and evaluating the expression status of specific biological substances based on a stained image obtained by producing a specimen slide using tissues and cells collected from the patient and staining it by a predetermined method.

In pathological diagnosis, widely used is a method of diagnosing the prognosis of breast cancer patients and predicting the therapeutic effect of a molecular target therapeutic agent "trastuzumab" (trade name "Herceptin (registered trademark), anti-HER2 monoclonal antibody) by quantifying and evaluating HER2 gene (HER2/neu, c-erbB-2), one type of oncogene, and/or HER2 protein, a membrane protein produced by HER2 gene, using a sample produced from a collected tumor tissue. The HER2 protein is thought to form a homodimer or a heterodimer bound to activated EGFR (HER1), HER3, or HER4, and signal, and is presumed to function as a receptor for a cancer cell growth factor (so far, no endogenous ligand that binds to HER2 is known).

Examples of the test methods for HER2 in a tumor tissue include immunohistochemical (IHC) method in which HER2 protein is stained, and fluorescent in situ hybridization (FISH) in which HER2 gene is stained. For test procedures and criteria (scores), ASCO/CAP HER2 test guideline (established in 2007, revised in 2013) issued by American Society of Clinical Oncology and American Society of Pathology is widely used in various countries, and also in Japan, HER2 test guide based on the revised ASCO/CAP HER2 test guideline (HER2 test guide fourth edition, breast cancer HER2 test pathology committee, April 2014) is used.

In the IHC method, generally used is a method in which an enzyme-labeled antibody is contacted with a sample, a protein to be detected and an antibody are directly or indirectly bound using an antigen-antibody reaction, and then a substrate corresponding to the enzyme is reacted to obtain a color. For example, DAB staining method in which peroxidase is used as an enzyme and diaminobenzidine is used as a substrate is widely used.

However, because, in the staining method based on the reaction between the enzyme and the substrate such as DAB staining method, color density largely varies depending on conditions such as temperature and time, there is a problem that accurately estimating the actual amount of the target protein from the color density is difficult.

Thus, in recent years, in order to label a gene or a protein, a method in which nano-sized particles in which a plurality of phosphors such as organic fluorescent dyes and semiconductor nanoparticles (quantum dots) is integrated, that is, phosper integrated particles (PID, which may be referred to as "fluorescent substance-integrated nanoparticles" or the like) are used has been proposed and developed for actual use. By labeling a target protein or gene with phosphor integrated particles and radiating excitation light adapted to the phosphor, the labeled protein or gene can be observed as a bright spot having a high brightness, and the location where the protein or gene is expressed and the amount thereof can be accurately evaluated. The phosphor integrated particles have an advantage that relatively long observation and imaging can be achieved because the phosphor integrated particles are less likely to discolor than a conventionally used staining agent.

As a method of fluorescently labeling a protein contained in a sample using phosphor integrated particles, the following method is known. For example, in Example of Patent Literature 1 (WO 2014/136885), a fluorescence labeling method (avidin-biotin combined secondary antibody method) is disclosed, in which phosphor integrated particles (dye resin particles) whose surfaces are modified with streptavidin are produced, a primary antibody (an anti-HER2 rabbit monoclonal antibody) is bound to an antigen (HER2 protein) on a tissue section, then a secondary antibody (an anti-rabbit IgG antibody) labeled with biotin is bound to the primary antibody, and then the streptavidin-modified phosphor integrated particles are bound to the secondary antibody.

The streptavidin-modified phosphor integrated particles are produced by the following method.
(i) Resin particles in which fluorescent dyes are integrated (contained) are formed using a melamine resin as a base, then the melamine resin is reacted with 3-aminopropyltrimethoxysilane, a silane coupling agent, to introduce an amino group to the particle surface, the amino group is further reacted with succinimidyl-[(N-maleimidopropionamido)-dodecaethylene glycol] ester as a linker to introduce a maleimide group to the surface of the melamine resin particles.
(ii) Streptavidin is reacted with N-succinimidyl S-acetylthioacetic acid to introduce a thiol group into streptavidin.
(iii) The maleimide group of the melamine resin particles of (i) above is reacted with the thiol group of streptavidin of (ii) above to bind them together.

In Patent Literature 2 (WO 2016/129444), disclosed are antibody-bound phosphor integrated nanoparticles in which a suitable number of antibodies are bound per unit area of the particle surface by reacting an antibody in which a suitable number of disulfide bonds (-S-S-) are reduced to produce a thiol group (-SH) by treatment with a specific reducing agent with phosphor integrated nanoparticles in which a ligand (for example, a maleimide group) capable of reacting with the thiol group is introduced onto the surface. The antibody may be a primary antibody used in a direct method of IHC method, or may be a secondary antibody or the like used in an indirect method.

For example, in Example of Patent Literature 2, an embodiment is disclosed in which HER2 is immunostained using antibody-modified fluorescent dye-containing silica nanoparticles produced by the following method.
(i) Fluorescent dye-containing silica nanoparticles are formed using 3-aminopropyltrimethoxysilane as a raw material and 5-carboxytetramethylrhodamine (registered trademark "TAMRA"), a fluorescent dye, and are reacted with succinimidyl-[(N-maleimidopropionamide) -dodecaethylene glycol] ester (product name "SM (PEG) ₁₂") as a linker to introduce a maleimide group onto the particle surface.
(ii) A primary antibody (an anti-HER2 rabbit monoclonal antibody) or a secondary antibody (an anti-rabbit IgG antibody) is treated with 2-mercaptoethanol or the like as a reducing agent to reduce the disulfide bond and produce a thiol groups.
(iii) The maleimide group of the silica nanoparticles of (i) above is reacted with the thiol group of the primary antibody or secondary antibody of (ii) above to bind them together.

### Citation List

### Patent Literature

Patent Literature 1: WO 2014/136885
Patent Literature 2: WO 2016/129444

### Summary of Invention

### Technical Problem

Though by the conventional immunostaining method with the phosphor integrated particles, as described in Patent Literatures 1 and 2 and the like, the target protein contained in a sample can be fluorescently labeled with a certain accuracy, the binding property of the phosphor integrated particles to the target protein is insufficient, and the sensitivity, that is, the target protein may not be detected with sufficient accuracy in some cases depending on the staining conditions, and the staining property need to be further improved.

In one embodiment of the present invention, it is an object to provide a means for improving the sensitivity as compared to that in the prior art in a method of fluorescently labeling a target protein contained in a sample using phosphor integrated particles.

### Solution to Problem

As a result of intensive investigations, the present inventors have found that the object can be achieved according to the following configuration example, thereby completing the present invention.

The configuration example of the present invention is as follows.
[1] Fluorescent premix particles, including: phosphor integrated particles that are modified with a first reactive substance; and at least one target protein-binding substance that is modified with a second reactive substance and is selected from the group consisting of an antibody and an aptamer, wherein the phosphor integrated particles and the at least one target protein-binding substance are linked by interaction between the first reactive substance and the second reactive substance.
[2] The fluorescent premix particles according to [1], wherein the first reactive substance is streptavidin and the second reactive substance is biotin.
[3] The fluorescent premix particles according to [1] or [2], wherein the target protein-binding substance is a primary antibody or a primary aptamer that specifically binds to a target protein.
[4] The fluorescent premix particles according to [1] or [2], wherein the target protein-binding substance is a secondary antibody or a secondary aptamer that specifically binds to a primary antibody that specifically binds to a target protein.
[5] The fluorescent premix particles according to any one of [1] to [4], wherein the phosphor integrated particles are particles including a resin base and a phosphor.
[6] The fluorescent premix particles according to any one of [1] to [5], wherein the phosphor integrated particles have a number of the target protein-binding substances linked per unit area of a surface of the phosphor integrated particles of 0.003 to 0.012 target protein-binding substances/nm².
[7] A fluorescent stain containing the fluorescent premix particles according to any one of [1] to [6].
[8] The fluorescent stain according to [7], wherein the fluorescent stain contains two or more types of the fluorescent premix particles having peaks of emission wavelength that are separated from each other by 100 nm or more and is a one-liquid type or a multi-liquid type.
[9] A fluorescent staining method in which a fluorescent stain containing the fluorescent premix particles according to [3] is used, the method including:
   binding the fluorescent premix particles in the fluorescent stain to a target protein.
[10] A fluorescent staining method in which a fluorescent stain containing the fluorescent premix particles according to [4] is used, the method including:
   binding the primary antibody to a target protein; and
   binding the fluorescent premix particles in the fluorescent stain to the primary antibody bound to the target protein.
[11] A fluorescent staining method in which the fluorescent stain according to [8] is used, the method including:
   directly or indirectly binding each of the fluorescent premix particles in the fluorescent stain that corresponds to each of two or more types of target proteins to the each of two or more types of target proteins.

### Advantageous Effects of Invention

According to one embodiment of the present invention, the sensitivity in fluorescently labeling a target protein can be significantly improved compared to conventional methods and the accuracy in quantification of the target protein can be improved.

### Brief Description of Drawings

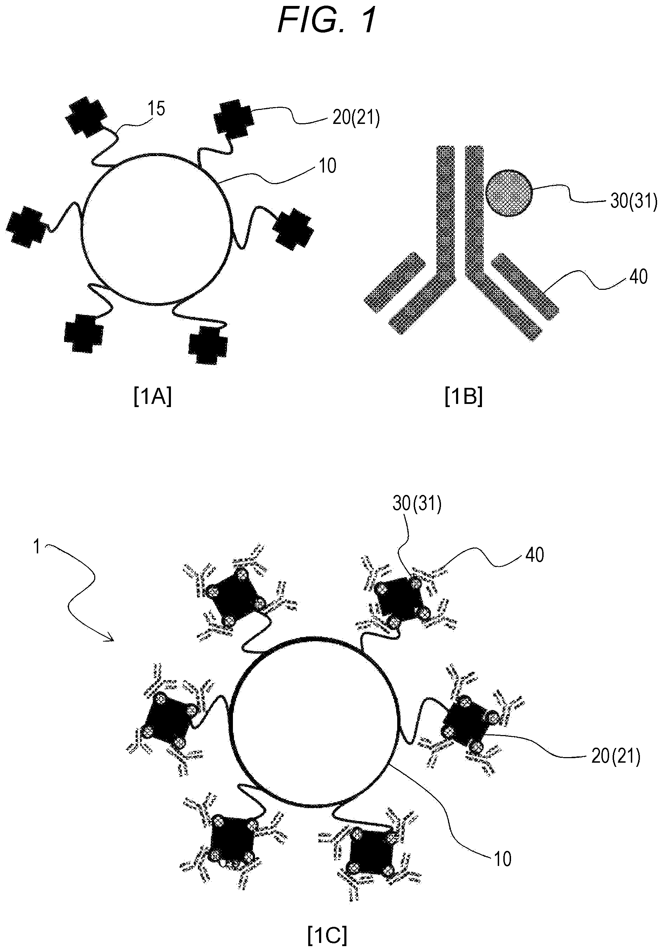

Fig. 1 is a schematic diagram showing a fluorescent premix particle in one embodiment of the present invention. [1A] indicates a phosphor integrated particle (10) modified with a first reactive substance (20), for example streptavidin (21), via a linker (15), [1B] indicates a target protein-binding substance (an antibody or aptamer) (40) modified with a second reactive substance (30), for example, biotin (31), and [1C] indicates a fluorescent premix particle (1) formed by linking between the first reactive substance (20) in [1A] above and the second reactive substance (30) in [1B] above by their interaction.

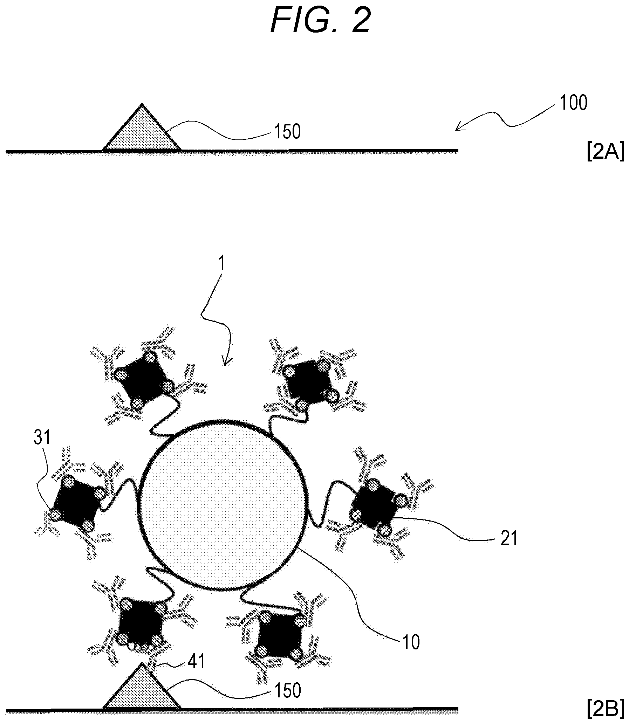

Fig. 2 is a schematic diagram showing a procedure in one embodiment (primary reaction staining method) of a fluorescent staining method of the present invention in which fluorescent premix particles to which a primary antibody is linked are used. [2A] indicates a state before start of a staining step where a target protein (150) on a tissue section (100) is unreacted, and [2B] indicates a step of binding a fluorescent premix particle (1) to which a primary antibody (41) is linked to the target protein (150).

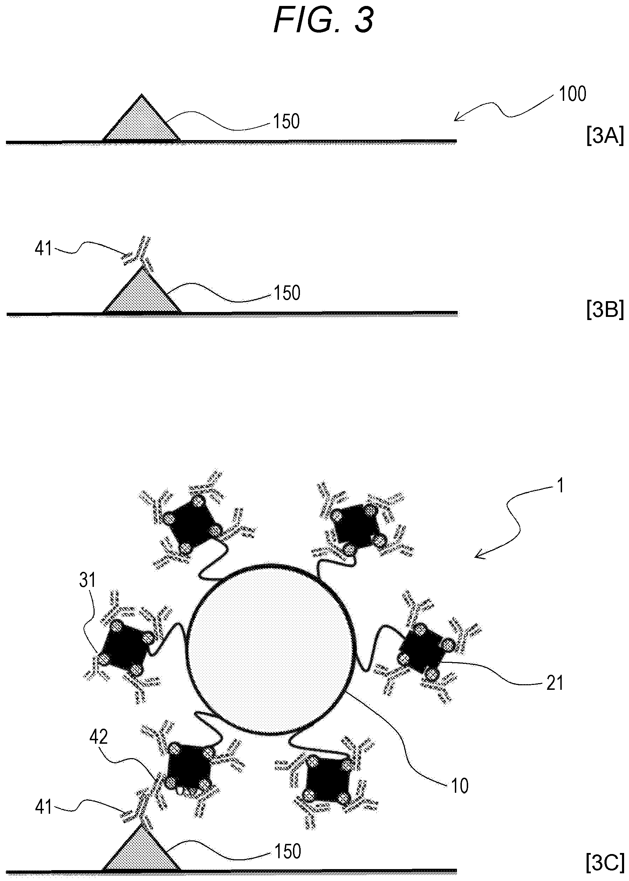

Fig. 3 is a schematic diagram showing a procedure in one embodiment (secondary reaction staining method) of a fluorescent staining method of the present invention in which fluorescent premix particles to which a secondary antibody is linked are used. [3A] indicates a state before start of a staining step where a target protein (150) on a tissue section (100) is unreacted, [3B] indicates a step of binding a primary antibody (41) to the target protein (150), and [3C] indicates a step of binding a fluorescent premix particle (1) to which a secondary antibody (42) is linked to the primary antibody (41).

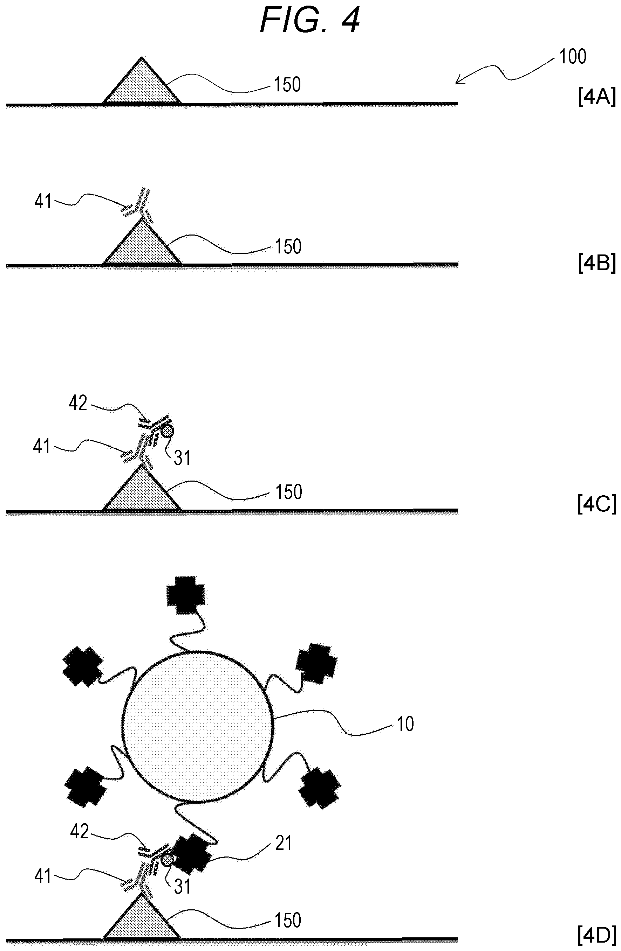

Fig. 4 is a schematic diagram showing a procedure of one embodiment of a fluorescent staining method according to a conventional biotin-avidin combined secondary antibody method. [4A] indicates a state before start of a staining step where a target protein (150) on a tissue section (100) is unreacted, [4B] indicates a step of binding a primary antibody (41) to a target protein (150), [4C] indicates a step of binding a secondary antibody (42) modified with biotin (31) to the primary antibody (41), and [4D] indicates a step of binding a phosphor integrated particle (10) modified with streptavidin (21) to the biotin (31).

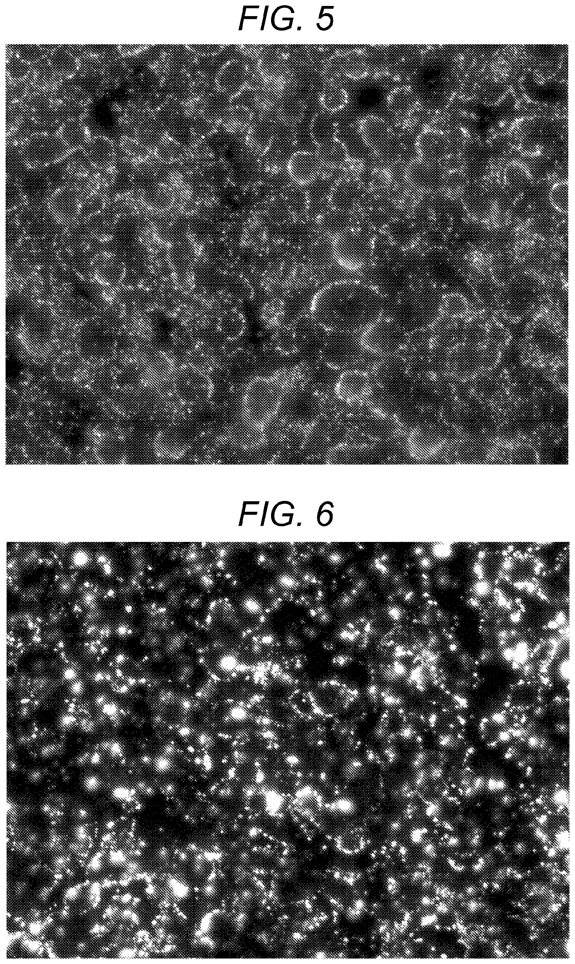

Fig. 5 is a fluorescently-stained image photographed in Comparative Example 1.
Fig. 6 is a fluorescently-stained image photographed in Example 1.

### Description of Embodiments

### -Fluorescent premix particles-

The fluorescent premix particles according to one embodiment of the present invention is a complex including: phosphor integrated particles that are modified with a first reactive substance; and at least one target protein-binding substance that is modified with a second reactive substance and is selected from the group consisting of an antibody and an aptamer, wherein the phosphor integrated particles and the at least one target protein-binding substance are linked by interaction between the first reactive substance and the second reactive substance.

The present inventors have found that the sensitivity of fluorescent labeling is significantly improved by altering, for example, a conventional fluorescent staining method such as the embodiment (avidin-biotin combined secondary antibody method) disclosed in Patent Literature 1 described above to a method in which streptavidin-modified phosphor integrated particles and biotin-labeled primary or secondary target protein-binding substance are reacted (premixed) in advance to prepare a complex, and the complex is bound to a target protein contained in a sample (for example, a tissue section), or an primary antibody is bound to a target protein and then the complex is bound to the primary antibody.

The followings are thought to be the reason.

Due to premixing, the binding efficiency between the first reactive substance and the second reactive substance becomes excellent.

When streptavidin is used as a first reactive substance and biotin is used as a second reactive substance, up to 4 biotin-labeled target protein-binding substances can be bound to one molecule of streptavidin on the surface of the phosphor integrated particles (because up to 4 molecules of biotin bind to one molecule of streptavidin), thus the phosphor integrated particles can carry an extremely large number of target protein-binding substances, and the binding efficiency to a target protein or a primary antibody is significantly increased.

The "fluorescent premix particles" are usually contained in a fluorescent stain, in other words, are used to produce a fluorescent stain. Because the fluorescent premix particles themselves function as a "fluorescent stain" that binds to a target protein and fluorescently labels it, the target protein-binding substance linked to the fluorescent premix particles before binding to the target protein is in an unreacted state.

Meanwhile, in a conventional immunostaining method such as an embodiment in which a primary antibody is reacted with a target protein, a biotin-modified secondary antibody is reacted with the primary antibody, and further streptavidin-modified phosphor integrated nanoparticles are reacted (biotin-avidin combined secondary antibody method), the particles are not fluorescent premix particles because the secondary antibody reacts with the primary antibody.

In the fluorescent premix particles, one phosphor integrated particle preferably has a suitable number of a target protein-binding substance linked on the surface of the phosphor integrated particle. Specifically, the phosphor integrated particles preferably have a number of the target protein-binding substances linked per unit area of a surface of the phosphor integrated particles of 0.003 target protein-binding substances/nm² or more, and more preferably 0.003 target protein-binding substances/nm² or more and 0.012 target protein-binding substances/nm² or less. Phosphor integrated particles to which surface target protein-binding substances within the appropriate or the preferable number range are linked can be produced by adjusting various conditions in the production method of fluorescent premix particles as described later (for example, an embodiment including the first to third steps).

The number of the target protein-binding substances linked per unit area of the surface of the phosphor integrated particles can be determined as follows, for example, by applying the method described in WO 2015/159776:
(i) The number per unit area of the first reactive substance introduced to the surface of the phosphor integrated particles is measured;
(ii) The coefficient showing the maximum number of the second reactive substance (the target protein-binding substance modified with the second reactive substance) that binds to one first reactive substance above is determined (for example, when the first reactive substance is avidin and the like, the coefficient is 4, and when the first reactive substance is an anti-hapten antibody, the coefficient is 1);
(iii) The value obtained by multiplying the number per unit area in (i) above by the coefficient in (ii) above is considered as the maximum value of the number of the target protein-binding substances that can be linked per unit area of the surface of the phosphor integrated particles; and
(iv) When the amount of the target protein-binding substance that is modified with the second reactive substance added for the reaction is larger than the maximum value of (iii), the maximum value is considered as the number of the target protein-binding substance per unit surface area, and when the amount is less than the maximum value of (iii), a value obtained by dividing the total added number of the target protein-binding substance modified with the second reactive substance by the total surface area of the phosphor integrated particles is considered as the number per unit surface area of the target protein-binding substance.

### «First reactive substance and second reactive substance»

The first reactive substance and the second reactive substance are a combination of substances that specifically bind each other by interaction, and both are selected from substances that do not intervene the specific binding between the target protein-binding substance and the target protein to which the target protein-binding substance binds (or the secondary, tertiary or higher level target protein-binding substance). Though the first reactive substance and the second reactive substance are not particularly limited, these can be selected from, for example, those used for indirectly binding a target protein (such as an antigen) and a labeling substance (such as a phosphor) in a known staining method.

For example, according to the immunostaining method (ABC method) in which an avidin-biotin complex is used, avidins such as avidin, streptavidin, and NeutrAvidin, preferably streptavidin, can be selected as the first reactive substance, and biotin can be selected as the second reactive substance. In such an embodiment, four biotins bind to one avidin, and thus the target biological substance can be detected with high sensitivity. In particular, it is particularly preferable because more target protein-binding substances can be linked to one phosphor integrated particle.

Instead of the combination of avidins and biotin, a hapten (a relatively low molecular weight substance that has antigenicity, but does not have immunogenicity, and is capable of reacting with an antibody) and an anti-hapten antibody can be used as the first reactive substance and the second reactive substance.

Examples of the combination of a hapten and an anti-hapten antibody include dicoxigenin and an anti-dicoxigenin antibody, FITC (fluorescein isothiocyanate) and an anti-FITC antibody, and DNP (2,4-dinitrophenyl) and an anti-DNP antibody.

### «Target protein-binding substance»

The target protein-binding substance is at least one selected from the group consisting of an antibody and an aptamer, and is a substance capable of binding directly or indirectly to a target protein.

The target protein-binding substance can be two or more types, but is usually one type.

### <Antibody>

The antibody is not particularly limited as long as it is capable of directly or indirectly binding to a target protein in the immunostaining method. Though the antibody can be a primary antibody that specifically binds to a target protein (an antigen), a secondary antibody that specifically binds to the primary antibody, or a higher level antibody, it is preferably a primary antibody or a secondary antibody.

The primary antibody is an antibody that recognizes and binds to an epitope unique to the antigen, and from the viewpoint of the stability of quantification of the target protein, a monoclonal antibody is more preferable than a polyclonal antibody. When two or more types of monoclonal antibodies are mixed and used, a combination of monoclonal antibodies that each specifically bind to different epitopes is preferable.

The immune animal that produces the primary antibody is not particularly limited, and can be selected from common animals, and examples of the common animals include a mouse, a rat, a guinea pig, a rabbit, a goat, and a sheep.

The secondary antibody or higher level antibody is an antibody that recognizes and binds to an epitope unique to the primary antibody or lower level antibody, preferably an epitope that exists in a region (Fc and the like) that is not involved in the binding of the antibody to the target protein (the antigen) and the like. Though the secondary antibody and the like are preferably a monoclonal antibody from the viewpoint of the stability of quantification of the target protein, a polyclonal antibody can be used from the economical viewpoint.

The immune animal that produces a secondary antibody and the like can be appropriately selected from among the animal species and the like exemplified as an immune animal that produces a primary antibody depending on the animal species that produces the primary antibody and the like or the animal species that forms a region such as Fc. For example, when a natural mouse antibody (an IgG produced by a mouse) is used as the primary antibody, an antibody that specifically binds to the mouse IgG and is produced by an immune animal other than a mouse (such as a rabbit) is appropriately used as a secondary antibody.

The antibody can be of any isotype, but is usually an IgG or an IgM, and in particular, an IgG is preferable. The antibody can be a natural antibody such as a full-length IgG, an antibody fragment such as Fab, Fab , F(ab)₂, Fv, and scFv, or a non-natural antibody such as an artificial antibody that is made multifunctional (multivalent or multispecific) using these antibody fragments as long as the antibody has the ability to specifically recognize and bind the target protein or lower antibody. The antibody can be a natural antibody derived from a certain immune animal (for example, a mouse antibody produced by a mouse), or can be a chimeric antibody or a humanized antibody produced by an artificial means using a vector and the like, or a complete human antibody.

### <Aptamer>

The aptamer is not particularly limited as long as the aptamer is capable of specifically labeling a target protein by a method according to an immunostaining method (direct method or indirect method) with an antibody. Though the aptamer can be a primary aptamer that specifically binds to a target protein, a secondary aptamer that specifically binds to a primary antibody or primary aptamer bound to a target protein, or a higher level aptamer, the aptamer is preferably a primary aptamer or secondary aptamer.

Aptamers can be roughly classified into nucleic acid (DNA or RNA) aptamers and peptide aptamers, and both nucleic acid aptamers and peptide aptamers can be used as long as they have the ability to specifically recognize and bind a target protein, an antibody bound to a target protein or a lower level aptamer.

Nucleic acid aptamers and peptide aptamers can be produced by a known method.

### <Phosphor integrated particles>

Though the phosphor integrated particles (particles before being modified with a first reactive substance) are not particularly limited, the phosphor integrated particles are preferably nano-sized particles (having a diameter of 1 µm or less) that have a structure in which a plurality of phosphors (for example, fluorescent dyes) is fixed and integrated in the inside or on the surface of organic or inorganic base particles, and are preferably particles in which one particle can emit fluorescence with sufficient brightness.

Such phosphor integrated particles are preferably used in fluorescent staining because the phosphor integrated particles have higher fluorescence intensity (brightness) in labeling the target protein, higher distinguishability from noises including cell auto fluorescence and other dyes, and less deterioration by radiation of excitation light (stronger light resistance) compared to single use of a phosphor (in one molecule without integration).

Though the phosphor that is integrated in the phosphor integrated particles is not particularly limited, for example, various known organic fluorescent dye molecules or semiconductor nanoparticles (which may be referred to as quantum dots and the like) can be used. Hereinafter, the phosphor integrated particles in which an organic fluorescent dye is used as a phosphor are called organic fluorescent dye integrated particles, and the phosphor integrated particles in which semiconductor nanoparticles are used as a phosphor are called inorganic phosphor integrated particles.

The phosphor used for the production of the phosphor integrated particles can be selected from phosphors that emit fluorescence of a desired wavelength (color) depending on the desired use. When two or more types of target proteins are fluorescently labeled, a combination of phosphors that emit fluorescence of different wavelengths corresponding to each protein is selected, and phosphor integrated particles in which each type of phosphors are integrated can be produced. When such two or more types of phosphors are used, phosphors that have peaks of emission wavelength that are separated from each other by 100 nm or more are preferably selected.

### (1) Organic fluorescent dye integrated particles

The organic fluorescent dye integrated particles are preferably nano-sized fluorescent particles in which a plurality of organic fluorescent dyes is integrated in the inside or on the surface of a base substance of the particles.

Examples of the organic fluorescent dye include fluorescent dyes that are composed of a low molecular weight organic compound (other than high molecular weight organic compound such as a polymer) such as a fluorescein dye, a rhodamine dye, Alexa Fluor (registered trademark, manufactured by Invitrogen) dye, BODIPY (registered trademark, manufactured by Invitrogen) dye, Cascade (registered trademark, manufactured by Invitrogen) dye, a coumarin dye, a NBD (registered trademark) dye, a pyrene dye, a cyanine dye, a perylene dye, an oxazine dye, and a pyrromethene dye. Among these, a rhodamine dye such as sulforhodamine 101 and Texas Red (registered trademark), which is the hydrochloride of sulforhodamine 101, a perylene dye such as perylene diimide, and a Pyrromethene dye such as Pyrromethene 556 are preferable because of their relatively high light resistance.

Examples of the base that constitutes the organic fluorescent dye integrated particles include substances capable of integrating organic fluorescent dyes with physical or chemical bonding force such as a resin and silica, and a resin is preferable. Specific examples thereof include resins such as polystyrene, polyamide, polylactic acid, polyacrylonitrile, polyglycidyl methacrylate, polymelamine, polyurea, polybenzoguanamine, polyfuran, polyxylene, phenolic resin, and ASA resin (acrylonitrile-styrene-methyl acrylate copolymer); polysaccharides; silica; and substances capable of stably integrating organic fluorescent dyes. Hydrophobic compounds such as polystyrene, polymelamine and silica, in particular melamine resin and styrene resin are preferable because fluorescent dye integrated particles can be easily produced from them and particles having high emission intensity can be obtained.

For example, organic fluorescent dye integrated particles that are produced using a fluorescent dye such as perylene diimide, sulforhodamine 101 or its hydrochloride (Texas Red), and Pyrromethene as a phosphor and a resin such as a melamine resin and a styrene resin as a base are preferable as the phosphor integrated particles because of the excellent labeling performance and the like.

### (2) Inorganic phosphor integrated particles

The inorganic phosphor integrated particles are preferably nano-sized fluorescent particles in which a plurality of semiconductor nanoparticles is integrated in the inside or on the surface of a base substance of the particles.

The semiconductor nanoparticles are not particularly limited, and examples thereof include quantum dots containing a Group II-VI compound, a Group III-V compound, or a Group IV element, and particle dots such as CdSe exemplified in WO 2012/133047.

Quantum dots in which semiconductor nanoparticles are used as a core and a shell is provided around the core can be also used. Hereinafter, as a notation of the semiconductor nanoparticles having shells, the semiconductor nanoparticles are described as CdSe/ZnS when a core is CdSe and a shell is ZnS.

Specific examples of the semiconductor nanoparticles having shells include CdSe/ZnS exemplified in WO 2012/133047.

The semiconductor nanoparticles can be surface-treated with an organic polymer or the like as needed. For example, CdSe/ZnS (manufactured by Invitrogen) whose particle surface is modified with a carboxy group, CdSe/ZnS (manufactured by Invitrogen) whose particle surface is modified with an amino group, or the like can be used.

Examples of the base that constitutes the inorganic phosphor integrated particles include substances capable of integrating semiconductor nanoparticles with physical or chemical bonding force such as a resin and silica, and a resin is preferable. Examples of the resin include thermosetting resins such as melamine resin, urea resin, benzoguanamine resin, phenol resin, and xylene resin; and various homopolymers and copolymers produced using one type, or two or more types of monomers such as styrene resin, (meth) acrylic resin, polyacrylonitrile, AS resin (acrylonitrile-styrene copolymer), and ASA resin (acrylonitrile-styrene-methyl acrylate copolymer).

The organic fluorescent dye integrated particle and the inorganic phosphor integrated body are known, and please see, for example, WO 2013/035703, WO 2013/147081, WO 2014/136776 and the like for the details and specific examples of embodiments of the phosphor and the base used for the production and the production method and the like.

### [Average particle size of phosphor integrated particles]

The average particle size of the phosphor integrated particles is preferably 30 to 300 nm, more preferably 40 to 160 nm. In general, the smaller the particle size, the larger the specific surface area, and the bonding force to a sample increases. However, when the average particle size is less than 30 nm, the bright spots to be observed in fluorescence observation due to the phosphor integrated particles may not be observed at all, or may be poorly observed. Meanwhile, when the average particle size of the phosphor integrated particles is more than 300 nm, for example, the bright spots observed in the fluorescence observation are too many, and the bright spots may not be separated each other and not be accurately counted.

Though the coefficient of variation indicating the variation of the particle size of the phosphor integrated particles is not particularly limited, it is preferably about 20% or less.

The particle size of the phosphor integrated particles can be measured as follows: an image is photographed using a scanning electron microscope (SEM), the cross section of resin particles for fluorescent labeling is measured, and the diameter (area equivalent circle diameter) when the measured value is considered as the area of the corresponding circle is taken as the particle size. The particle size of each of the phosphor integrated particles contained in a group having a sufficient number (for example, 1000) is measured as described above, then the arithmetic average is calculated and taken as the average particle size, and the coefficient of variation is calculated by the formula: 100 × standard deviation of particle size / average particle size.

By adjusting the conditions in the production, the average particle size of the phosphor integrated particles falls within a desired range.

One example of the production method of organic fluorescent dye integrated particles is the emulsion polymerization method, specifically, a method in which a phosphors is added while (co)polymerizing a monomer for synthesizing a resin (thermoplastic resin or thermosetting resin), a base of the particles, and the phosphor is taken in the inside or on the surface of the (co)polymer. In the reaction system in the emulsion polymerization method, micelles having an aqueous phase in the outer side and an oil phase in the inner side are formed by the surfactant, monomers that constitute the resin are contained in the oil phase in the inner side of the micelles, and in this inner side of the micelles, polymerization reaction occurs. In the synthesis of organic fluorescent dye integrated particles by such emulsion polymerization method, for example, by adding a surfactant having an emulsifying action in an amount of 10 to 60% by weight relative to the resin raw material, particles having an average particle size of 30 to 300 nm can be produced. When the amount of surfactant used is constant, also by changing the rate of each of the resin raw material and the phosphor used in the production of the fluorescent dye integrated particles in the total reaction system, the average particle size of the fluorescent dye integrated particles can be adjusted.

Meanwhile, for example, by, after the production of the inorganic phosphor integrated particles, classifying the particles by the size selective precipitation method and collecting a fraction of the inorganic phosphor integrated particles having a predetermined particle size, the average particle size of the inorganic phosphor integrated particles falls within a predetermined range.

The size selective precipitation method is a method in which an adsorbent having a lipophilic group is preliminary adsorbed onto the surface of the inorganic phosphor integrated particles, then the inorganic phosphor integrated particles are dispersed in a lipophilic solvent, and an amphiphilic additive is gradually added to the solvent to precipitate the particles. Because the dispersibility of the inorganic phosphor integrated particles strongly depends on the interaction between the adsorption group on the particle surface and the solvent, by gradually adding the additive, aggregation precipitates are formed in order from the inorganic phosphor integrated particles of the larger size. The precipitates are collected by centrifugation, and redispersed in a solvent to obtain inorganic phosphor integrated particles having a narrow particle size distribution.

Examples of the adsorbent having a lipophilic group include compounds having an alkyl group such as heptane, octane, and dodecane, and compounds having 8 to 12 carbon atoms are preferable.

Examples of the lipophilic solvent include pyridine and hexane, and as the amphiphilic additive, chloroform, methanol and the like are preferably used.

Examples of the lipophilic group that can be adsorbed on the surface of inorganic phosphor integrated particles such as quantum dots include a phosphino group such as trioctylphosphine (TOP), a phosphine oxide such as trioctylphosphine oxide (TOPOT), a phosphate group, and an amino group.

### <Production method of fluorescent premix particles>

The production method of the fluorescent premix particles is not particularly limited as long as the fluorescent premix particles have a structure including: phosphor integrated particles that are modified with a first reactive substance; and a target protein-binding substance that is modified with a second reactive substance, wherein the phosphor integrated particles and the target protein-binding substance are linked by interaction between the first reactive substance and the second reactive substance.

In general, the fluorescent premix particles can be produced by producing each of the phosphor integrated particles that are modified with a first reactive substance and the target protein-binding substance that is modified with a second reactive substance, and then mixing them in an appropriate dispersion medium, for example, PBS, and preferably the fluorescent premix particles can be produced by the following first to third steps.

### - First step: preparation step of the phosphor integrated particles that are modified with a first reactive substance

The first step is a step for preparing the phosphor integrated particles that are modified with a first reactive substance (hereinafter also referred to as "first reactive substance-modified phosphor integrated particles"). This step can be the same embodiment as the step for preparing the phosphor integrated particles that are modified with streptavidin and the like (the first reactive substance in the present invention), which is used in the conventional immunostaining method with an avidin-biotin complex (ABC method).

The first reactive substance can be produced according to a known method. The reactive site of the synthesized phosphor integrated particles can be directly linked to the first reactive substance by covalent bond or the like, or indirectly, specifically, using a linker having reactive functional groups at both ends, the reactive functional groups of the linker can be each reacted with the reactive site of phosphor integrated particles and the reactive site of the first reactive substance to form a covalent bond, thereby the phosphor integrated particles and the first reactive substance are linked via the linker.

Examples of the reactive site of the first reactive substance include an amino group, a carboxy group, and a thiol group, which are contained in common proteins. The reactive site can be a site originally contained in the first reactive substance, or a site introduced into the first reactive substance using a treating agent (a compound) other than a linker (for example, introduced onto the particle surface by the reaction with a compound such as a silane coupling agent). For example, when the first reactive substance is a protein such as streptavidin, examples of the method of introducing a thiol group include a method in which N-succinimidyl S-acetylthioacetate (SATA) is reacted, then deprotection with hydroxylamine is performed to introduce a thiol group into the amino group originally contained in the first reactive substance, and a method in which the disulfide bond (-S-S-) originally contained in the first reactive substance is cleaved by treatment with a reducing agent such as dithiothreitol (DTT) to produce a thiol group. When such a thiol group is used as a reactive site contained in the first reactive substance, the reactive functional group to be contained in the linker to bind the phosphor integrated particles and the first reactive substance together is preferably a group capable of reacting with a thiol group, for example, a maleimide group. The linker can have such a reactive functional group capable of reacting with the reactive site of the first reactive substance at one end or both ends, and the reactive functional groups at both ends can be the same or different. The linker can be a compound having a chain structure such as a polyoxyalkylene moiety, typically, a PEG (polyethylene glycol) chain in the molecule. The function of the linker can be performed by the silane coupling agent itself as described below.

When particles whose base is silica are used as the phosphor integrated particles, a silanol group on the surface of the silica particle can be used as a reactive site contained in the phosphor integrated particles (silica particles), or a reactive site other than a silanol group that is introduced onto the surface of the silica particles using a silane coupling agent can be used.

Examples of the silane coupling agent used herein include a compound and the like having a hydrolysable group such as an alkoxy group, an acetoxy group, and a halo group at one end, and having a functional group such as an amino group, a mercapto group, and an epoxy group at the other end. The hydrolysable group in the silane coupling agent is converted to a silanol group by a hydrolysis reaction and then causes a condensation reaction with the silanol group on the surface of the silica particles (or the silanol group in other silane coupling agents), thereby a functional group such as amino group can be introduced onto the surface of the silica particles.

The introduced functional group such as an amino group itself can be used to react with the reactive site contained in the first reactive substance, or the introduced functional group can be further reacted with a reactive functional group at one end of the linker to react the reactive functional group at the other end of the linker with the reactive site contained in the first reactive substance as needed. For example, when a mercapto group or amino group introduced by a silane coupling agent is used as a reactive site contained in the phosphor integrated particles, a linker having a maleimide group or N-hydroxysuccinimide (NHS) group at one end is preferably used as the reactive functional group that reacts with the reactive site.

When particles whose base is a resin are used as the phosphor integrated particles, the reactive site contained in the phosphor integrated particles (resin particles) can be a functional group that has been contained in the raw material (a monomer and the like) used for synthesizing the resin and is remaining even after the synthesis, or can be a predetermined structure formed by synthesis reaction of the resin, or can be a site that is introduced to the reactive site originally contained in the phosphor integrated particles using a treating agent (a compound) other than the linker.

For example, when phosphor integrated particles whose base is a melamine resin are used, by reacting a silane coupling agent having a hydrolysable group (for example, trialkoxy group) to be adsorbed to the melamine resin at one end and an amino group on the other end, or by reacting a linker having amino groups at both ends, an amino group as the reactive functional group that is reactive with the reactive site contained in the first reactive substance can be introduced onto the surface of the melamine resin particles. In the linker having amino groups at both ends, one of the amino groups of the linker reacts with a reactive site contained in the melamine resin, for example, a methylol group (-CH₂OH) of methylolmelamine produced when melamine and formaldehyde, which are used for the synthesis of melamine resin, are reacted in advance, or a reactive functional group for the etherified (-CH₂OR) produced by further reaction of the methylol group with alcohol.

When phosphor integrated particles whose base is a styrene resin are used, by using a monomer capable of copolymerizing with styrene and having a functional group such as an amino group or an epoxy group in the side chain in the synthesis of the styrene resin, styrene resin particles having a functional group such as an amino group and an epoxy group on the surface as the reactive site to react with the reactive functional group at one end of the linker are obtained.

For the linker and silane coupling agent for the reaction as described above, those having various reactive functional groups at both ends are commercially available and can be easily obtained, and those having desired reactive functional groups at both ends can be also obtained by synthesis according to a known method. For example, as a linker having an NHS group (a reactive functional group capable of reacting with an amino group) at one end of a PEG chain and a maleimide group (a reactive functional group capable of reacting with a thiol group) at the other end, a product such as "SM(PEG)ₙ" (n = 2, 4, 6, 8, 12, 24) (manufactured by Thermo Fisher Scientific) and the like can be used.

The linker can be reacted with the first reactive substance and/or the phosphor integrated particles according to a known protocol. The modification state of the phosphor integrated particles by the first reactive substance may vary depending on reaction conditions of the linker with the first reactive substance and/or the phosphor integrated particles, for example, the number (density) of reactive sites contained in the first reactive substance and/or the phosphor integrated particles, the ratio of the number of molecules of the linker to the number of molecules of the first reactive substance and/or the phosphor integrated particles used for the reaction (concentration and volume of each solution), the type and used amount of the reaction reagent, reaction temperature, reaction time and the like, and thus these conditions can be appropriately adjusted.

In the first step, the reaction of the linker with the first reactive substance and the reaction of the linker with the phosphor integrated particles can be performed sequentially (or simultaneously if possible). For example, the phosphor integrated particles can be first bound to the linker, and then the linker bound to the phosphor integrated particles at one end can be bound to the first reactive substance.

After the first step, centrifugation, purification with a column, and washing are preferably performed to remove unreacted substances and impurities as needed, and then the first reactive substance-modified phosphor integrated particles (dispersion liquid) are collected and used in the third step.

### - Second step: preparation step of the target protein-binding substance that is modified with a second reactive substance

The second step is a step for preparing the target protein-binding substance that is modified with a second reactive substance (hereinafter also referred to as "second reactive substance-modified target protein-binding substance"). Typically, using a linker having reactive functional groups at both ends, each reactive functional group of the linker is reacted with the reactive site contained in the target protein-binding substance and the reactive site contained in the second reactive substance to form a covalent bond, thereby the target protein-binding substance and the second reactive substance are linked via a linker. For example, when biotin is used as the second reactive substance, this step can be the same embodiment as the step for preparing the antibody that is modified with biotin, which is used in the conventional immunostaining method with an avidin-biotin complex (ABC method).

As the linker for the second step, the same linker as that in the first step described above can be used. Due to the commonality of the reaction of the linker with a protein, the reaction mode of the linker with the second reactive substance (preferably biotin) and the reaction mode of the linker with the target protein-binding substance can be the same as the reaction mode of the linker with the first reactive substance (preferably streptavidin) in the first step described above, and can be modified as needed.

A linker having biotin attached at one end in advance and a reactive functional group for a reactive site contained in the target protein-binding substance (for example, a maleimide group for a thiol group) at the other end is commercially available as a product such as a so-called biotin labeling kit including necessary reaction reagents and the like, and is commonly used. When such a kit is used, the necessary reaction in the second step is only the reaction to bind the biotinylated linker to the target protein-binding substance.

In the second step, the reaction of the linker with the second reactive substance (which is not necessary when a product such as the kit described above is used), and the reaction of the linker with the target protein-binding substance can be performed sequentially (or simultaneously if possible).

After the reaction of the second step, conventional centrifugation, purification with a column, washing and the like are performed as needed, and then the second reactive substance-modified target protein-binding substance is collected and used in the third step.

### - Third step: reaction step between the first reactive substance-modified phosphor integrated particles and the second reactive substance-modified target protein-binding substance

The third step is a step of finally producing fluorescent premix particles by reacting the first reactive substance-modified phosphor integrated particles prepared in the first step with the second reactive substance-modified target protein-binding substance prepared in the second step.

This third step can be performed by mixing the first reactive substance-modified phosphor integrated particles and the second reactive substance-modified target protein-binding substance in an appropriate solvent (for example, in a buffer such as PBS) and reacting them for a predetermined time.

After the third step, centrifugation, purification with a column, and washing are preferably performed to remove unreacted substances and impurities as needed, and then the fluorescent premix particles are collected and dispersed in an appropriate solvent to obtain a fluorescent stain.

For the purification, size separation column treatment, gel filtration column treatment, adsorption separation column treatment and the like, which are suitable for removing molecules, proteins and the like smaller than particles, can be used. DOJIN IgG purification kit -A/G is an adsorption separation column and can be used appropriately.

### -Fluorescent stain-

The fluorescent stain according to one embodiment of the present invention contains the fluorescent premix particles. A fluorescent stain can be generally prepared as a dispersion liquid obtained by preparing and collecting fluorescent premix particles, and then dispersing them in an appropriate dispersion medium, for example PBS (phosphate buffered saline) containing 1% BSA.

When the fluorescent stain is used in an embodiment in which two or more types of target proteins are targets for fluorescent labeling, the fluorescent stain can contain two or more types of fluorescent premix particles that correspond to each target protein. In such a case, the two or more types of fluorescent premix particles preferably have peaks of the fluorescence wavelengths that are sufficiently separated from each other, for example, preferably separated by 100 nm or more so that the distinguishability of the fluorescence (bright spots) of the fluorescent premix particles that label each target protein will not be adversely affected. Such a fluorescent stain used to target more than one target proteins can be a one-liquid type in which two or more types of fluorescent premix particles are contained in the same pack (dispersion liquid), or a multi-liquid type in which each type of fluorescent premix particles is contained in separate packs. According to the embodiment of the staining method, the fluorescent stain can contain, in addition to the one-liquid type or multi-liquid type pack of fluorescent premix particles, a pack of other reagents (for example, a stain for cell morphology observation).

### -Fluorescent staining method-

Though the fluorescent stain can be used in various fluorescent staining methods, typically, it is used to produce a stained slide in which a target protein contained in a sample is fluorescently labeled by immunostaining from a tissue slide produced from a tissue section, a sample.

Basic embodiments of the fluorescent staining method in which phosphor integrated particles are used and the analysis method such as pathological diagnosis in which the produced stained slide is used are known. In general, the fluorescent staining method can be performed by steps such as a specimen pretreatment step (deparaffinization, antigen activation, cell fixation, washing and the like), a staining step (immunostaining, staining for morphology observation, fluorescent premix particle treatment, blocking and the like), and a specimen post-treatment step (sealing, treatment for transparency, dehydration and the like). The analysis method in which the completed stained slide is used can be performed by steps such as an observation and photographing step, an image processing and analysis step in which the photographed image is used and the like. Such a fluorescent staining method and analysis method can be appropriately performed with reference to, for example, Patent Literatures such as WO 2013/035688, JP-A2015-117980, WO 2014/136885, WO 2016/129444, and WO 2015/163209, or according to general or known technical matters.

### «Staining step»

### (1) Immunostaining

Immunostaining is staining a sample (for example, a tissue section) to fluorescently label a target protein based on the immunostaining method, and the embodiment of the immunostaining is also changed depending on the embodiment of the fluorescent premix particles contained in the fluorescent stain to be used.

There are various immunostaining methods, and they are not particularly limited as long as they are capable of fluorescently labeling a target protein and staining a sample (a tissue section) for pathological diagnosis and the like. However, typical examples thereof include the following methods.

The first embodiment of the immunostaining (primary reaction staining method) is an embodiment in which a fluorescent stain containing fluorescent premix particles in which phosphor integrated particles and a primary antibody or primary aptamer are linked is used according to the immunostaining method (primary antibody method) in which the target protein is directly fluorescently labeled and stained, and the embodiment includes the step of binding fluorescent premix particles in the fluorescent stain to a target protein in the sample.

The second embodiment of immunostaining (secondary reaction staining method) is an embodiment in which a fluorescent stain containing fluorescent premix particles in which phosphor integrated particles and a secondary antibody or secondary aptamer are linked is used according to the immunostaining method (secondary antibody method) in which the target protein is indirectly fluorescently labeled and stained, and the embodiment includes the steps of binding a primary antibody or primary aptamer to a target protein in the sample, and binding fluorescent premix particles in a fluorescent stain to the primary aptamer or primary aptamer.

As the third embodiment (primary reaction multiple staining method) and the fourth embodiment (secondary reaction multiple staining method) of immunostaining, to which the two embodiments described above are applied, there is also an embodiment including the step of directly or indirectly binding each of fluorescent premix particles in a fluorescent stain that corresponds to each of two or more types of target proteins in the sample to the each of two or more types of target proteins in the sample. In this case, the peaks of the emission wavelengths of each of the fluorescent premix particles are separated from each other by 100 nm or more. The fluorescent stain can be a multi-liquid type including a set of fluorescent stains each containing one type of fluorescent premix particles, or a one-liquid type including one fluorescent stain containing all types of fluorescent premix particles.

Immunostaining can be performed according to known (ideally standardized) procedures and treatment conditions corresponding to embodiments as described above or other modified embodiments.

Generally, a sample slide having a sample (a tissue section) after the specimen pretreatment step placed thereon can be immersed in one type or two or more types of reagents (fluorescent stains and the like) according to the embodiment of immunostaining under appropriate conditions of temperature and time (for example, overnight at 4°C).

In the second or fourth embodiment, the sample slide can be first immersed in a solution containing a primary antibody, and then the obtained sample slide can be immersed in a fluorescent stain containing fluorescent premix particles to which a secondary antibody or a secondary aptamer is linked.

In the third or fourth embodiment, when a fluorescent stain of a multi-liquid type is used, the sample slide can be immersed in each fluorescent stain sequentially.

The reagent necessary for immunostaining can be produced according to a known method or is commercially available.

The stained sample can be washed using a washing solution such as PBS as needed between staining steps included in immunostaining or after the whole immunostaining. For example, the sample slide can be washed by immersing the sample slide in PBS at room temperature for 3 to 30 minutes, and the PBS can be changed during the immersion as needed.

### (2) Staining for cell morphology observation

Staining for cell morphology observation is usually performed after immunostaining, and is a treatment for staining the sample (a tissue section) with a stain for morphology observation to observe the shape of cells or tissues and obtain positional information of each part of cells.

Examples of the stain for morphology observation typically include a stain including a dye such as hematoxylin stain, an eosin stain, a papanicolaou (Pap) stain. Staining with such a dye can be performed according to a general procedure. For example, when hematoxylin-eosin (HE) staining is performed, for example, the sample can be stained with Mayers hematoxylin solution for 5 minutes, washed with running water at 45°C for 3 minutes, and then stained with 1% eosin solution.

As a stain for morphology observation, a fluorescent stain including phosphor integrated particles can also be used. For example, as described in WO 2015/146896, a biological substance that is constitutively expressed in the cell membrane of a sample and is other than the target protein (hereinafter also referred to as "reference biological substance") can be observed as bright spots of phosphor integrated particles labeling the reference biological substance on the cell membrane by fluorescently staining the sample using phosphor integrated particles. The region where such bright spots are integrated is in the cell membrane where the reference biological substance is expressed or in the vicinity thereof (cell membrane region), and thus the shape of cells or tissues can be observed and the positional information of cells can be obtained. Such a treatment for fluorescently labeling a reference biological substance and the solution used for the treatment are also considered to be one embodiment of staining and a fluorescent stain. The fluorescent premix particles can be used to immunostain a reference biological substance.

The reference biological substance can be appropriately selected from among biological substances that are uniformly and constitutively expressed on the cell membrane and are expressed in a sufficient amount to achieve the purpose of morphology observation. For example, membrane proteins such as ATPase, cadherin, cytokeratin, EpCAM (Epithelial Cell Adhesion/Activating Molecule: also referred to as epithelial cell adhesion molecule, CD326, KSA, or TROP1) can be a preferable reference biological substance.

The staining agent for fluorescently labeling the reference biological substance by fluorescent staining preferably includes phosphor integrated particles bound to a substance that specifically binds to the reference biological substance.

As the substance that specifically binds to the reference biological substance, an antibody that specifically recognizes and binds a protein as a reference biological substance as an antigen can be used. For example, when cadherin is a reference biological substance, an anti-cadherin antibody can be used.

### (3) Fixation of fluorescent premix particles

The fixation of fluorescent premix particles is performed using a predetermined fixation reagent after immunostaining and before other treatments such as staining for morphology observation, and is a treatment of fixing the target protein labeled with fluorescent premix particles on the sample (the tissue section). When a reference biological substance is a target of fluorescent staining for the staining for cell morphology observation, the same fixation can be performed after the treatment and before other treatments.

Examples of the fixation reagent include a reagent capable of crosslinking fluorescent premix particles bound to the target protein with the target protein or a protein in the vicinity thereof.

Thus, it is presumed that, by performing the fixation of fluorescent premix particles, the detachment of fluorescent premix particles from the target protein and the release from the vicinity of the target protein be prevented, and the problem of the decreased number of fluorescent bright spots of fluorescent premix particles in observing and photographing the stained slide be solved even when the sample (the tissue section) is immersed in various solutions for staining for morphology observation, blocking, washing and the like.

### (Fixation reagent)

Examples of the fixation reagent for the fluorescent premix particles include a compound (a crosslinker) having reaction sites (hereinafter referred to as "third reaction site" and "fourth reaction site", respectively) capable of reacting with each of a reaction site present on the surface of the fluorescent premix particles (hereinafter referred to as a "first reaction site") and a reaction site contained in the protein in the vicinity of the sample (the tissue section) (hereinafter referred to as a "second reactive site").

Examples of the first reaction site and the second reaction site include functional groups such as an amino group (ε-amino group of the lysine side chain, α-amino group at N terminal), a thiol group, a hydroxy group, a carboxy group, and an aromatic. The first reaction site and the second reaction site can be the same or different.

Meanwhile, examples of the third reaction site and the fourth reaction site include functional groups such as an NHS (N-hydroxysuccinimide) group, a maleimide group, an aldehyde group, an epoxy group, and an iodine group. The third reaction site and the fourth reaction site can be the same or different, and one functional group can function as both the third reaction site and the fourth reaction site (for example, an aldehyde group when formaldehyde is used as a fixation reagent). The third reaction site and the fourth reaction site can be linked by a linker such as PEG. A plurality of third reaction sites and/or fourth reaction sites can exist in one molecule of the fixation reagent.

The fixation of fluorescent premix particles can be performed by contacting the fixation reagent solution at an appropriate concentration with the sample (the tissue section) for an appropriate time. Though the concentration of the fixation reagent in the fixation reagent solution can be appropriately adjusted, it is preferably, for example, 1 to 500 µM. In particular, when a fixation reagent to which functional groups are attached at both ends via a linker such as PEG is used, the crosslinking reaction may not proceed sufficiently at too high concentration. That is, only the functional group at one end of the molecule of the fixation reagent may bind to the protein in the vicinity of the sample (the tissue section) or fluorescent premix particles, and the other functional group at the opposite side of the linker may remain without reacting with the crosslinking partner (another molecule of the fixation reagent has already bound to the crosslinking partner). When the fluorescent premix particles and the sample (the tissue section) are covered with excessive fixation reagent, the intensity of the fluorescence emitted from the fluorescent premix particles may be reduced, thus the distinguishability may be reduced, the reaction between the stain used in staining for morphology observation and the target may be prevented, and the staining by these treatments may become insufficient.

The contact time (the fixation time) between the fixation reagent solution and the sample can also be appropriately adjusted, and it is, for example, about several minutes to several hours at room temperature.

### (4) Blocking

Blocking is performed, for example, before and after the antigen-antibody reaction in immunostaining, and examples thereof include a treatment for preventing the nonspecific adsorption of fluorescent premix particles to the target protein, and the adsorption of avidins on the surface of the fluorescent premix particles (unreacted sites in premixing) to the endogenous biotin present in the living body.

The endogenous biotin is abundantly present in the liver, kidney, muscle, mammary gland, and digestive tract in humans, and may not be inactivated by cryopreservation of tissue sections and cell fixation in the specimen production step (depending on conditions of the type of the fixation reagent used and the like). Thus, when a section derived from any of the above-mentioned tissues such as mammary gland is stained, blocking for the biotin-avidin reaction is preferably performed.

For example, when a method according to the secondary antibody method is used as an immunostaining method, blocking for antigen-antibody reaction to suppress the nonspecific adsorption of a primary antibody (a primary aptamer), a secondary antibody (a secondary aptamer) or the like to proteins other than the target predetermined antigen and the like can be performed before the treatment of binding a primary antibody or a primary aptamer to a target protein (primary antibody treatment), or before the treatment of binding fluorescent premix particles (fluorescent premix particle treatment) to which a secondary antibody or a secondary aptamer is linked to the primary antibody or the primary aptamer, and further, in the case where a reference biological substance is used for fluorescent staining for staining for morphology observation, before the treatment of binding the primary antibody to the reference biological substance, or before the treatment of binding the fluorescently labeled secondary antibody to the primary antibody.

Blocking can be performed using a known blocking agent. For example, BSA-containing PBS buffer can be used for blocking for antigen-antibody reaction, and a blocking reagent (for example, "Endogenous avidin-biotin blocking kit" manufactured by NICHIREI BIOSCIENCES INC.) containing a avidin solution (treatment solution for blocking the binding of unreacted sites of four reaction sites of avidin and the like contained in fluorescent premix particles by binding to endogenous biotin), a biotin solution (treatment solution for blocking unreacted sites of four reaction sites of avidin and the like contained in fluorescent premix particles by binding of biotin to the unreacted sites, and blocking the binding to endogenous biotin) and the like can be used for blocking for avidin-biotin reaction. When staining is performed more than once to the same sample (the tissue section) as in multiple staining for multiple target proteins, blocking and the fluorescence premix fixation prior to that can be performed more than once as needed.

### «Target protein»

The target protein, the target of the fluorescent staining, is preferably at least one biological substance contained in the sample, and further, is most preferably a protein (an antigen) that is a target of immunostaining performed for quantification or detection of a protein mainly in pathological diagnosis. Typically, for example, the target protein is preferably a protein related to pathological diagnosis of cancer (a so-called biomarker). Specific examples thereof include receptors for a growth factor such as PD-L1 (Programmed cell death1 ligand 1), CTLA4 (cytotoxic T lymphocyte antigen-4), CD8, CD30, CD48, CD59, or EGFR (HER1) (Epidermal Growth Factor Receptor), HER2 (Human Epidermal Growth Factor Receptor), HER3, HER4, VEGFR (Vasular Endothelial Growth Factor Receptor), IGFR (Insulin-like Growth Factor Receptor), and HGFR (Hepatocyte Growth Factor Receptor), and proteins that are receptors for immune system such as PD-1 (Programmed cell death 1), an important inhibitory immune checkpoint molecule on the surface of T cells, which is the receptor for PD-L1 above.

### Examples

Hereinafter, though the present invention will be described in detail by way of Examples, the present invention is not limited to these Examples.

The summary of Production Examples, Examples, and Comparative Examples is shown in the following tables.

**[Table 1]**

| | Fluorescent stain | Reaction liquid 1 | | | | Reaction liquid 2 | | | Column purification |
|---|---|---|---|---|---|---|---|---|---|
| | | Phosphor integrated particles | | First reactive substance | Amount of reaction liquid | Target protein-binding substance | Second reactive substance | Amount of reaction liquid | |
| | | Fluorescent dye | Base | | | | | | |
| Example 1 (Production Example 4, 8) | No. 1 | Red | Silica | SA | 25 µL | Anti-HER2 antibody | Biotin | 25 µL | No |
| | | (TA) | | | | (primary antibody) | | | |
| Example 2 (Production Example 4, 8) | No. 2 | Red | Silica | SA | 10 µL | Anti-HER2 antibody | Biotin | 90 µL | N o |
| | | (TA) | | | | (primary antibody) | | | |
| Example 3 (Production Example 4, 8) | No. 3 | Red | Silica | SA | 95 µL | Anti-HER2 antibody | Biotin | 5 µL | N o |
| | | (TA) | | | | (primary antibody) | | | |
| Example 4 (Production Example 4, 8) | No. 4 | Red | Silica | SA | 98 µL | Anti-HER2 antibody | Biotin | 2 µL | No |
| | | (TA) | | | | (primary antibody) | | | |
| Example 5 (Production Example 4, 8) | No. 5 | Red | Silica | SA | 25 µL | Anti-HER2 antibody | Biotin | 25 µL | Yes |
| | | (TA) | | | | (primary antibody) | | | |
| Example 6 (Production Example 4, 10) | No. 6 | Red | Silica | SA | 25 µL | HER2 antigen-recognizing RNA aptamer | Biotin | 25 µL | No |
| | | (TA) | | | | (primary aptamer) | | | |
| Example 7 (Production Example 5, 11) | No. 7 | Red | Melamine resin | SA | 25 µL | Rabbit IgG-recognizing RNA aptamer | Biotin | 25 µL | No |
| | | (TA) | | | | (secondary aptamer) | | | |
| Example 8 (doublestaining) (Production Example 6, 12/7, 13) | No. 8 | Green | Melamine resin | Anti-DNP antibody | 10 µL | Anti-mouse IgG antibody | DNP | 90 µL | No |
| | | (PM) | | | | | | | |
| | | Red | Silica | Anti-DNP antibody | 10 µL | Anti-rabbit IgG antibody | FITC | 90 µL | N o |
| | | (TA) | | | | | | | |

| | | | | | | | | | |
|---|---|---|---|---|---|---|---|---|---|
| TA: Texas Red PM: Pyrromethene 556 SA: Streptavidin DNP: Dinitrophenyl | | | | | | | | | |

**[Table 2]**

| | Primary reaction | Secondary reaction | Conventional fluorescent labeling reaction | Average brightness in fluorescently-stained image |
|---|---|---|---|---|
| Example 1 | Anti-HER2 antibody-modified phosphor integrated particles | | | 612 |
| | (fluorescent stain: No. 1) | | | |
| Example 2 | Anti-HER2 antibody-modified phosphor integrated particles | | | 688 |
| | (fluorescent stain: No. 2) | | | |
| Example 3 | Anti-HER2 antibody-modified phosphor integrated particles | | | 630 |
| | (fluorescent stain: No. 3) | | | |
| Example 4 | Anti-HER2 antibody-modified phosphor integrated particles | | | 472 |
| | (fluorescent stain: No. 4) | | | |
| Example 5 | Anti-HER2 antibody-modified phosphor integrated particles | | | 540 |
| | (fluorescent stain: No. 5) | | | |
| Comparative Example 1 | Anti-HER2 rabbit antibody | Biotin-modified anti-rabbit IgG antibody | Streptavidin-modified Texas Red integrated silica particles | 141 |
| | | (Production Example 9) | | |
| | | | (Production Example 4) | |
| Example 6 | HER2 antigen-recognizing RNA aptamer-modified phosphor integrated particles | | | 1701 |
| | (fluorescent stain: No. 6) | | | |
| Example 7 | Anti-HER2 rabbit antibody | Rabbit IgG-recognizing RNA aptamer-modified phosphor integrated particles (fluorescent stain: No. 7) | | 1920 |
| Example 8 (doublestaining) | Anti-PD-L1 mouse antibody | Anti-mouse IgG antibody-modified phosphor integrated particles (Production Example 6, 12: DIG/anti-DIG antibody) | | |
| | Anti-EGFR rabbit antibody | Anti-rabbit IgG antibody-modified phosphor integrated particles (Production Example 7, 13: FITC/anti- FITC antibody) | | |

### [Production Example 1] Production of Texas Red integrated silica particles

"Texas Red-X" (Sulforhodamine 101-X, manufactured by Sigma-Aldrich Co. LLC) (3.4 mg), a red organic fluorescent dye and 3-aminopropyltrimethoxysilane (manufactured by Shin-Etsu Silicone, KBM 903) (3 µL) were mixed in N,N-dimethylformamide (DMF) to obtain an organoalkoxysilane compound.

The obtained organoalkoxysilane compound (0.6 mL) was mixed with 99% ethanol (48 mL), tetraethoxysilane (TEOS) (0.6 mL), ultrapure water (2 mL), and 28% by mass of ammonia water (2.0 mL) at 5°C for 3 hours.

The mixed liquid produced in the above step was centrifuged at 10000 G for 20 minutes, and the supernatant was removed. To this precipitate, ethanol was added to disperse the precipitate, and the resulting liquid was centrifuged again for washing. Further, the same washing was repeated twice to obtain Texas Red integrated silica particles (excitation wavelength: 590 nm, emission wavelength: 620 nm). One thousand of the obtained particles were observed with a SEM, the particle size was measured, and the average particle size was calculated and found to be 160 nm.

### [Production Example 2] Production of Texas Red integrated melamine resin particles

"Texas Red" (Sulforhodamine 101-X, manufactured by Sigma-Aldrich Co. LLC) (5.25 mg), a red organic fluorescent dye was dissolved in 22.5 mL of pure water, and then the resulting solution was stirred for 20 minutes while maintaining the temperature of the solution at 70°C with a hot stirrer. To the solution after stirring, 0.21 g of a melamine resin raw material "Nikalac MX-035" (manufactured by NIPPON CARBIDE INDUSTRIES CO.,INC.) was added, and the mixture was further heated and stirred under the same conditions for 5 minutes.

To the resulting solution, 680 µL of a 10% aqueous solution of dodecylbenzenesulfonic acid (manufactured by KANTO KAGAKU) was added as a reaction initiator, and the mixture was heated and stirred at 70°C for 50 minutes. Then, the mixture was heated to 90°C, heated and stirred for 20 minutes, and then the solution was allowed to stand and cool to room temperature. To remove impurities such as the excessive resin raw material and organic fluorescent dyes from the obtained dispersion liquid of Texas Red integrated melamine resin particles, the solution after cooling was dispensed into centrifuge tubes and centrifuged at 12,000 rpm for 20 minutes to precipitate the Texas Red integrated melamine resin particles contained in the solution. The supernatant was removed, and the precipitated particles were washed with ethanol and water to obtain Texas Red integrated melamine resin particles.

One thousand of the obtained particles were observed with a SEM, the particle size was measured, and the average particle size was calculated and found to be 40 nm.

### [Production Example 3] Production of Pyrromethene 556 integrated melamine resin particles

"Pyrromethene 556" (14.4 mg), a green organic fluorescent dye, was added to 22 mL of water and dissolved. Then, 2 mL of a 5% aqueous solution of EMULGEN (registered trademark) 430 (polyoxyethylene oleyl ether, manufactured by Kao Corporation), an emulsifier for emulsion polymerization, was added to this solution. The solution was heated to 70°C while stirring on a hot stirrer, and then 0.65 g of a melamine resin raw material "Nikalac MX-035" (manufactured by NIPPON CARBIDE INDUSTRIES CO.,INC.) was added thereto.

Further, to this solution, 1000 µL of a 10% aqueous solution of dodecylbenzenesulfonic acid (manufactured by KANTO KAGAKU) was added as a reaction initiator, and the mixture was heated and stirred at 70°C for 50 minutes. Then, the mixture was heated to 90°C, and heated and stirred for 20 minutes.

To remove impurities such as the excessive resin raw material and fluorescent dyes from the obtained dispersion liquid, the dispersion liquid was washed with pure water. Specifically, the dispersion liquid was centrifuged at 20000 G for 15 minutes in a centrifuge (Micro Refrigerated Centrifuge 3740, manufactured by KUBOTA Corporation co., ltd.), the supernatant was removed, then ultrapure water was added thereto, and the resulting mixture was sonicated to be redispersed. Washing by centrifugation, supernatant removal and redispersion in ultrapure water was repeated 5 times.

By the above-described treatment, Pyrromethene 556 integrated melamine resin particles (excitation wavelength: 490 nm, emission wavelength: 520 nm) were produced. One thousand of the obtained particles were observed with a SEM, the particle size was measured, and the average particle size was calculated and found to be 155 nm.

### [Production Example 4] Production of streptavidin-modified Texas Red integrated silica particles

The Texas Red integrated silica particles obtained in Production Example 1 were adjusted to a concentration of 3 nM using PBS containing 2 mM EDTA (ethylenediaminetetraacetic acid), to the obtained liquid, SM(PEG)₁₂ (succinimidyl-[(N-maleimidopropionamide)-dodecane ethylene glycol] ester, manufactured by Thermo Scientific) was added as a linker to the final concentration of 10 mM, and the mixture was mixed and reacted at 5°C for 1 hour.

The obtained reaction liquid was centrifuged at 10000 G for 20 minutes, and the supernatant was removed. PBS containing 2 mM EDTA was added thereto to disperse the precipitate, and the resulting liquid was centrifuged again under the same condition. Washing under the same condition was repeated 3 times to obtain maleimide group-introduced Texas Red integrated silica particles.

Meanwhile, 40 µL of an aqueous solution of streptavidin (manufactured by Wako Pure Chemical Industries, Ltd.) adjusted to 1 mg/mL was added to 210 µL of borate buffer, then 70 µL of 2-iminothiolane hydrochloride (manufactured by Sigma-Aldrich Co. LLC) adjusted to 64 mg/mL was added thereto, and the mixture was reacted at room temperature for 1 hour. Thereby, a mercapto group was introduced to the amino group of streptavidin (-NH-C(=NH²⁺Cl⁻)-CH₂-CH₂-CH₂-SH). The obtained solution was desalted through a gel filtration column (Zaba Spin Desalting Columns: manufactured by Funakoshi Co., Ltd.) to obtain mercapto group-introduced streptavidin capable of binding to maleimide group-introduced Texas Red integrated silica particles.

Using PBS containing 2 mM EDTA, 740 µL of a liquid obtained by mixing the total amount (0.04 mg) of the obtained mercapto group-introduced streptavidin and the maleimide group-introduced Texas Red integrated silica particles so that the concentration of the silica particles would be 0.67 nM was prepared and reacted at room temperature for 1 hour. Then, 10 mM mercaptoethanol was added thereto to stop the reaction.

The obtained liquid was concentrated by a centrifugal filter, and then unreacted substances were removed using a gel filtration column for purification to obtain streptavidin-modified Texas Red integrated silica particles. A purified streptavidin-modified Texas Red integrated silica particle dispersion liquid having a concentration of the particles adjusted to 0.02 nM was prepared using 50 mM Tris solution.

Measurement was performed according to the method described in WO 2015/159776, and it was presumed that the obtained streptavidin-modified Texas Red integrated silica particles have streptavidins linked to the surface of the Texas Red integrated silica particles at a density of 0.0311 streptavidins/nm².

### [Production Example 5] Production of streptavidin-modified Texas Red integrated melamine resin particles

The Texas Red integrated melamine resin particles (0.1 mg) obtained in Production Example 2 were dispersed in 1.5 mL of ethanol, 2 µL of aminopropyltrimethoxysilane "LS-3150" (manufactured by Shin-Etsu Chemical Co., Ltd.) was added thereto, and the mixture was reacted at room temperature for 8 hours with stirring to introduce an amino group on the particle surface.

The amino group-introduced Texas Red integrated melamine resin particles were adjusted to a concentration of 3 nM using PBS containing 2 mM EDTA, SM(PEG)₁₂ was added as a linker to a final concentration of 10 mM to the obtained liquid, and the mixture was mixed and reacted at room temperature for 1 hour with stirring.

The obtained reaction liquid was centrifuged at 10,000 G for 20 minutes, the supernatant was removed, then PBS containing 2 mM EDTA was added thereto to disperse the precipitate, and the resulting liquid was centrifuged again under the same condition. Washing under the same condition was repeated 3 times to obtain maleimide group-introduced Texas Red integrated melamine resin particles.

Meanwhile, to 40 µL of an aqueous solution of streptavidin (manufactured by Wako Pure Chemical Industries, Ltd.) adjusted to 1 mg/mL, 70 µL of an aqueous solution of N-succinimidyl-S-acetylthioacetate (SATA, manufactured by PIRCE) adjusted to 64 mg/mL was added, and the mixture was reacted at room temperature for 1 hour. Thereby, a protected mercapto group was introduced to the amino group of streptavidin (-NH-CO-CH₂-S-CO-CH₃). Subsequently, a deprotected (free) mercapto group was produced from the protected mercapto group by hydroxylamine treatment, thereby introduction of a mercapto group to streptavidin was completed. The obtained solution was desalted through a gel filtration column (Zaba Spin Desalting Columns: manufactured by Funakoshi Co., Ltd.) to obtain mercapto group-introduced streptavidin.

The total amount (0.04 mg) of mercapto group-introduced streptavidin produced as described above and maleimide group-introduced Texas Red integrated melamine resin particles were mixed in PBS containing 2 mM EDTA, the mixture was reacted for 1 hour, and then 10 mM mercaptoethanol was added to the reaction liquid to stop the reaction.

The obtained liquid was concentrated by a centrifugal filter, and then unreacted substances were removed using a gel filtration column for purification to collect purified streptavidin-modified Texas Red integrated melamine resin particles. A purified streptavidin-modified Texas Red integrated melamine resin particle dispersion liquid having a concentration adjusted to 0.02 nM was prepared using 50 mM Tris solution.

### [Production Example 6] Production of anti-DNP antibody-modified Pyrromethene 556 integrated melamine resin particles

A dispersion liquid of anti-DNP antibody-modified Pyrromethene 556 integrated melamine resin particles was prepared according to the same procedure as in Production Example 5 except that the Pyrromethene 556 integrated melamine resin particles obtained in Production Example 3 was used instead of the Texas Red integrated melamine resin particles obtained in Production Example 2 and an anti-DNP (dinitrophenyl) antibody was used instead of streptavidin.

### [Production Example 7] Production of anti-FITC antibody-modified Texas Red integrated silica particles

A dispersion liquid of anti-FITC antibody-modified Texas Red integrated silica particles was prepared according to the same procedure as in Production Example 4 except that an anti-FITC antibody was used instead of streptavidin.

### [Production Example 8] Production of biotin-modified anti-HER2 antibody (biotin-modified primary antibody)

Anti-Erb 2 antibody [EP1045Y] (manufactured by Abcam plc.), a rabbit monoclonal antibody, was used as a primary antibody against a target protein HER2, that is, an anti-HER2 antibody. This anti-HER2 antibody was dissolved in 50 mM Tris solution to prepare a primary antibody solution.

Meanwhile, a linker reagent "maleimide-PEG₂-biotin" (manufactured by Thermo Scientific, product number 21901) was adjusted to 0.4 mM using DMSO. This linker reagent solution (8.5 µL) was added to the primary antibody solution, the mixture was mixed and reacted at 37°C for 30 minutes to bind biotin to the anti-HER2 antibody via a PEG chain. The obtained reaction solution was passed through a desalting column to purify the biotin-modified anti-HER2 antibody.

The concentration of the protein (the biotin-modified primary antibody) in the solution was calculated by measuring the absorbance at a wavelength of 300 nm of the purified biotin-modified primary antibody (biotin-modified anti-HER2 antibody) solution using a spectrophotometer ("F-7000" manufactured by Hitachi, Ltd.). The concentration of the biotin-modified primary antibody was adjusted to 6 µg/mL using 50 mM Tris solution to obtain a biotin-modified primary antibody solution.

### [Production Example 9] Production of biotin-modified anti-rabbit IgG antibody (secondary antibody)

An anti-rabbit IgG antibody "LO-RG1" (manufactured by GeneTex, cord GTX 40383) was used as a secondary antibody against the anti-HER2 antibody (rabbit monoclonal antibody). This anti-rabbit IgG antibody (50 µg) was dissolved in 50 mM Tris solution, reducing agent DTT (dithiothreitol) was added to this solution to a final concentration of 3 mM, the mixture was mixed and reacted at 37°C for 30 minutes to produce an anti-rabbit IgG antibody in which a disulfide bond was reduced to produce a mercapto group, that is, a reduced anti-rabbit IgG antibody. Then, the reaction solution was passed through a desalting column "Zeba Desalt Spin Columns" (manufactured by Thermo Scientific, Cat. #89882) to purify the reduced anti-rabbit IgG antibody.

In 50 mM Tris solution, 200 µL of the total amount of the purified reduced anti-rabbit IgG antibody was dissolved to prepare a secondary antibody solution.

Meanwhile, a linker reagent "maleimide-PEG₂-biotin" (manufactured by Thermo Scientific, product number 21901) was adjusted to 0.4 mM using DMSO to obtain a linker reagent solution. This linker reagent solution (8.5 µL) was added to the secondary antibody solution, the mixture was mixed and reacted at 37°C for 30 minutes to bind biotin to the anti-rabbit IgG antibody via a PEG chain. The obtained solution was passed through a desalting column to purify the biotin-modified anti-rabbit IgG antibody.

The concentration of the protein (the biotin-modified secondary antibody) in the solution was calculated by measuring the absorbance at a wavelength of 300 nm of the purified biotin-modified secondary antibody (biotin-modified anti-rabbit IgG antibody) solution using a spectrophotometer ("F-7000" manufactured by Hitachi, Ltd.). The concentration of the biotin-modified secondary antibody was adjusted to 6 µg/mL using 50 mM Tris solution to obtain a biotin-modified secondary antibody solution.

### [Production Example 10] Production of biotin-modified HER2 antigen-recognizing RNA aptamer

The HER2 antigen-recognizing RNA aptamer (RNA 5-AGCCGCGAGGGGAGGGAUAGGGUAGGGCGCGGCU-3, NUCLEIC ACID THERAPEUTICS, Volume 21, Number 3, 2011, 173) having 5 end modified with biotin was obtained by synthesis request to Hokkaido System Science Co., Ltd. to synthesize it.

The obtained biotin-modified HER2 antigen-recognizing RNA aptamer was adjusted to a concentration of 6 µg/mL using a 50 mM Tris solution to obtain a biotin-modified HER2 antigen-recognizing RNA aptamer solution.

### [Production Example 11] Production of biotin-modified rabbit IgG-recognizing RNA aptamer

The rabbit IgG-recognizing RNA aptamer (RNA 5-GGGAGAAUUCCGACCAGAAG-UUCGAUACGCCGUGGGGUGACGUUGGCUAC-CCUUUCCUCUCUCCUCCUUCUUC-3, Analytical Biochemistry, 375, 2008, 217-222) having 5 end modified with biotin was obtained by synthesis request to Hokkaido System Science Co., Ltd.

The obtained biotin-modified rabbit IgG-recognizing RNA aptamer was adjusted to a concentration of 6 µg/mL using 50 mM Tris solution to obtain a biotin-modified rabbit IgG-recognizing RNA aptamer solution.

### [Production Example 12] Production of DNP-modified anti-mouse IgG antibody

Using NHS-DNP reagent (BP-22397, manufactured by BroadPharm), the NHS-DNP group was reacted with the amino group of goat-derived anti-mouse IgG antibody "ab182017" (manufactured by Abcam plc.) to produce an DNP modified anti-mouse IgG antibody.

The concentration of the produced DNP-modified anti-mouse IgG antibody was adjusted to 6 µg/mL using 50 mM Tris solution to obtain a DNP-modified anti-mouse IgG antibody solution.

### [Production Example 13] Production of FITC-modified anti-rabbit IgG antibody

Using NHS-FITC reagent (BP-22401, BroadPharm), the NHS-FITC group was reacted with the amino group of rat-derived anti-rabbit IgG antibody "LO-RG1" (manufactured by GeneTex, Inc., cord GTX 40383) to produce FITC-modified anti-rabbit IgG antibody.

The concentration of the produced FITC-modified anti-rabbit IgG antibody was adjusted to 6 µg/mL using 50 mM Tris solution to obtain a FITC-modified anti-rabbit IgG antibody solution.

### [Comparative Example 1] Production of stained slide according to conventional avidin-biotin combined secondary antibody method

The anti-HER2 antibody used in Production Example 8 (the anti-HER2 antibody before modification with biotin in Production Example 8) was used as a primary antibody, the biotin-modified anti-rabbit IgG antibody obtained in Production Example 9 was used as a secondary antibody, and the streptavidin-modified Texas Red integrated silica particles obtained in Production Example 4 were used as a fluorescent stain. A specimen pretreatment step (deparaffinization, activation), a staining step (primary antibody treatment, secondary antibody treatment, fluorescent labeling, fixation of phosphor integrated particles, and staining for morphology observation), and a specimen post-treatment step (sealing) were performed according to the procedure as shown in (1), (2A), and (3) below to produce a stained slide based on immunostaining method (see Fig. 4). Then, using the produced stained slide, observation and imaging were performed according to the procedure shown in (4) below.

### (1) Specimen pretreatment step

### (1-1) Deparaffinization

Tissue array slide (CB-A712) (HER2 positive stained control specimen) manufactured by Cosmo Bio whose FISH score was previously calculated using PathVysion HER-2 DNA probe kit (Abbott) was used. The tissue array slide was deparaffinized according to the procedure of (i) to (iii) below.
(i) The tissue array slide was immersed in a container containing xylene at normal temperature for 30 minutes. During immersion, xylene was replaced 3 times.
(ii) The tissue array slide obtained in (i) was immersed in a container containing ethanol at normal temperature for 30 minutes. During immersion, ethanol was replaced 3 times.
(iii) The tissue array slide obtained in (ii) was immersed in a container containing water for 30 minutes. During immersion, water was replaced 3 times.

### (1-2) Activation

The deparaffinized tissue array slide was activated according to the procedure of (i) to (iv) below.
(i) Washing was performed to replace the solvent of the tissue array slide with water.
(ii) The tissue array slide obtained in (i) was placed in 10 mM citrate buffer (pH 6.0) and autoclaved at 121°C for 15 minutes.
(iii) The tissue array slide after autoclaving was immersed in a container containing PBS for 30 minutes and washed.
(iv) The tissue array slide obtained in (iii) was brought into contact with PBS containing 1% BSA for blocking for 1 hour.

### (2) Staining step

### (2-1A) Immunostaining

The activated (1-2) tissue array slide was immunostained according to the procedure of (i) to (iii) below.
(i) Primary antibody treatment: a 0.05 nM solution of Anti-Erb2 antibody [EP1045Y] (manufactured by Abcam plc.), rabbit monoclonal antibody, was dropped onto a tissue section on the tissue array slide using PBS containing 1% BSA, and those were reacted at 4°C overnight.
(ii) Secondary antibody treatment: the tissue array slide after the primary reaction was washed with PBS, then 6 µg/mL of biotin-modified anti-rabbit IgG antibody solution obtained in Production Example 9 was dropped onto the tissue section, and those were reacted at room temperature for 30 minutes.
(iii) Fluorescent labeling: the tissue array slide after the secondary reaction was washed with PBS, then 0.02 nM streptavidin-modified Texas Red integrated silica particles obtained in Production Example 4 was dropped onto the tissue section, and those were reacted at 4°C overnight.

### (2-2) Fixation of phosphor integrated particles

The tissue array slide after fluorescent labeling (2-1A) was immersed in an aqueous solution of 4% neutral paraformaldehyde for 10 minutes to perform fixation of phosphor integrated particles.

### (2-3) Staining for morphology observation

The tissue array slide after the fixation of phosphor integrated particles (2-2) was stained for morphology observation (HE staining, hematoxylin-eosin staining) according to the procedure of (i) to (ii) below.
(i) The tissue array slide after the fixation (2-2) was stained with Mayers hematoxylin solution for 5 minutes to perform hematoxylin staining. Then, the slide was washed with running water at 45°C for 3 minutes.
(ii) The tissue array slide after washing was stained with 1% eosin solution for 5 minutes to perform eosin staining, thereby a stained slide was produced.

### (3) Specimen post-treatment step

The stained slide after the staining for morphology observation (2-3) was sealed according to the procedure of (i) to (ii) below.
(i) ENTELLAN NEW (manufactured by Merck & Co.) was dropped onto the stained slide at normal temperature, then a cover glass was put on the slide, and the slide was air-dried at normal temperature for 10 minutes for sealing.
(ii) After (i), the stained slide after the sealing was protected from light and stored until observation and photographing of a stained image.

### (4) Observation

A predetermined excitation light (an excitation light having a wavelength corresponding to the excitation wavelength of Texas Red used as a fluorescent dye) was radiated to the stained slide after the sealing using a fluorescence microscope ("BX-53" manufactured by Olympus Corporation), emitted fluorescence was observed, and simultaneously a fluorescence staining image was photographed using a digital camera for a microscope ("DP73" manufactured by Olympus Corporation). The excitation light was set to 575 to 600 nm using an optical filter for excitation light. The range of the wavelength (nm) of fluorescence for observation was also set to 612 to 692 nm through an optical filter for fluorescence.

The conditions of the excitation wavelength during the microscope observation and image photographing were set so that the radiation energy in the vicinity of the visual field center at excitation of 580 nm would be 900 W/cm². The exposure time for image photographing was arbitrarily set (for example, set to 4000 µs) so that the brightness of the image would not be saturated. The maximum point of brightness was detected by image processing software ImageJ (open source) based on the image imaged at a magnification of 400 and was counted as a bright spot.

Next, an image stained by hematoxylin staining was photographed by observation in a bright field of a fluorescence microscope and image photographing. The image of fluorescent staining and the image of hematoxylin staining were overlaid together by image processing, and the number of bright spots per cell was counted. The number of bright spots was counted for 1000 cells, and the obtained average was taken as the number of bright spots in each tissue section.

The fluorescently-stained image photographed is shown in Fig. 5. The average brightness (brightness per pixel) of the obtained fluorescently-stained image was 141.

### [Example 1] Preparation of fluorescent stain (No. 1) containing anti-HER2 antibody-linked Texas Red integrated silica particles and photographing of fluorescently-stained image using the same

The 0.02 nM streptavidin-modified Texas Red integrated silica particle dispersion liquid (reaction liquid 1) obtained in Production Example 4 (25 µL) and the biotin-modified anti-HER2 antibody (a primary antibody) solution (reaction solution 2) at a concentration of 6 µg/mL obtained by Production Example 8 (25 µL) were mixed and reacted at room temperature for 1 hour to prepare a fluorescent stain (No. 1) containing anti-HER2 antibody (a primary antibody) -linked Texas Red integrated silica particles (fluorescent premix particles).

The obtained fluorescent premix particles were presumed to have an anti-HER2 antibody linked to the surface of Texas Red integrated silica particles at a density of 0.012 anti-HER2 antibodies/nm².

In Comparative Example 1, a stained slide was prepared in the same manner as in Comparative Example 1 except that the method in (2-1A) above was changed to the method in (2-1B) below (see Fig. 2), and the fluorescently-stained image was photographed. The fluorescently-stained image photographed is shown in Fig. 6. The average brightness of the obtained fluorescently-stained image was 612.

### (2-1B) Immunostaining

The activated (1-2) tissue array slide was immunostained according to the procedure below.

That is, the concentration of a fluorescent stain (No. 1) containing fluorescent premix particles, anti-HER2 antibody (a primary antibody) -linked Texas Red integrated silica particles was adjusted to a concentration of 0.02 nM using 50 mM Tris solution. This solution was dropped onto the tissue section on the tissue array slide and those are reacted at 4°C overnight.

### [Example 2] Preparation of fluorescent stain (No. 2) containing anti-HER2 antibody-linked Texas Red integrated silica particles and photographing of fluorescently-stained image using the same

The streptavidin-modified Texas Red integrated silica particle dispersion liquid at a concentration of 0.02 nM obtained in Production Example 4 (10 µL) and the biotin-modified anti-HER2 antibody (a primary antibody) solution at a concentration of 6 µg/mL obtained in Production Example 8 (90 µL) were mixed and reacted at room temperature for 1 hour to prepare a fluorescent stain (No. 2) containing anti-HER2 antibody (a primary antibody) - linked Texas Red integrated silica particles (fluorescent premix particles).

The obtained fluorescent premix particles were presumed to have an anti-HER2 antibody linked to the surface of Texas Red integrated silica particles at a density of 0.030 anti-HER2 antibodies/nm².

A stained slide was produced in the same manner as in Example 1 except that the obtained fluorescent stain (No. 2) was used, and the fluorescently-stained image was photographed. The average brightness of the obtained fluorescently-stained image was 688.

### [Example 3] Preparation of fluorescent stain (No. 3) containing anti-HER2 antibody-linked Texas Red integrated silica particles and photographing of fluorescently-stained image using the same

The streptavidin-modified Texas Red integrated silica particle dispersion liquid at a concentration of 0.02 nM obtained in Production Example 4 (95 µL) and the biotin-modified anti-HER2 antibody (a primary antibody) solution at a concentration of 6 µg/mL obtained in Production Example 8 (5 µL) were mixed and reacted at room temperature for 1 hour to prepare a fluorescent stain (No. 3) containing anti-HER2 antibody (a primary antibody) - linked Texas Red integrated silica particles (fluorescent premix particles).

The obtained fluorescent premix particles were presumed to have an anti-HER2 antibody linked to the surface of Texas Red integrated silica particles at a density of 0.003 anti-HER2 antibodies/nm².

A stained slide was produced in the same manner as in Example 1 except that the obtained fluorescent stain (No. 3) was used, and the fluorescently-stained image was photographed. The average brightness of the obtained fluorescently-stained image was 630.

### [Example 4] Preparation of fluorescent stain (No. 4) containing anti-HER2 antibody-linked Texas Red integrated silica particles and photographing of fluorescently-stained image using the same

The streptavidin-modified Texas Red integrated silica particle dispersion liquid at a concentration of 0.02 nM obtained in Production Example 4 (98 µL) and the biotin-modified anti-HER2 antibody (a primary antibody) solution at a concentration of 6 µg/mL obtained in Production Example 8 (2 µL) were mixed and reacted at room temperature for 1 hour to prepare a fluorescent stain (No. 4) containing anti-HER2 antibody (a primary antibody) - linked Texas Red integrated silica particles (fluorescent premix particles).

The obtained fluorescent premix particles were presumed to have an anti-HER2 antibody linked to the surface of Texas Red integrated silica particles at a density of 0.001 anti-HER2 antibodies/nm².

A stained slide was produced in the same manner as in Example 1 except that the obtained fluorescent stain (No. 4) was used, and the fluorescently-stained image was photographed. The average brightness of the obtained fluorescently-stained image was 472.

### [Example 5] Preparation of fluorescent stain (No. 5) containing purified anti-HER2 antibody-linked Texas Red integrated silica particles and photographing of fluorescently-stained image using the same

First, a fluorescent stain (No. 1) was prepared in the same manner as in Example 1.

Subsequently, the fluorescent stain (No. 1) was purified by the following procedure to prepare a fluorescent stain (No. 5).

### [Step A] Production of protein A-bound resin column

A dextran hydroxyl group of gel filtration carrier "Sephacryl S-1000 SF" (manufactured by GE Healthcare Japan Corporation, resin matrix in which allyl dextran and N, N-methylenebisacrylamide are crosslinked covalently) and bromoacetic acid were reacted for 16 hours to carboxymethylate the surface of the carrier (see Monchaux, E., and Vermette, P. (2008). Cell adhesion resistance mechanisms using arrays of dextran-derivative layers. J. Biomed Mater Res A 85, 1052-1063). "Sephacryl S-1000 SF" is a porous body having a pore that an object (liposome) having a particle diameter of 230 nm can enter and pass through (see http://lifesciencedb.jp/dbsearch/Literature/get_pne_cgpdf.php?year=1990&number=3511&file=2Qgovzb50x7RW4I cCUhKPw==).

Subsequently, a mixed liquid containing 1-ethyl-3-(3-dimethylaminopropyl) carbodiimide hydrochloride (EDC: manufactured by DOJINDO LABORATORIES) as water-soluble carbodiimide (WSC) at a concentration of 400 nM and N-hydroxysuccinimide (NHS: manufactured by Thermo Fisher Scientific) at a concentration of 100 mM was prepared, and the carboxymethylated carrier and the mixed liquid were reacted to convert the carboxyl group to the active ester. A solution of protein A ("21184" manufactured by Thermo Fisher Scientific) was further reacted with them, and the amino group contained in the protein A was bound to the active esterified carboxyl group by dehydration. The protein A-bound resin thus obtained was packed in a column (1 mL syringe) to produce a protein A-bound resin column.

### [Step B] Purification of anti-HER2 antibody-linked fluorescent premix particles

Buffer A (0.1 M glycine + 1.2 M sodium tartrate, pH 9.0) (5 mL) was flowed 3 times to equilibrate the protein A-bound resin column produced in Step A.

The fluorescent stain (No. 1) (1.5 mL) was diluted with buffer A (1.5 mL), and the diluted fluorescent stain was added to the equilibrated protein A-bound resin column to bind the anti-HER2 antibody (IgG) in fluorescent premix particles to the protein A in the protein A-bound resin column. The column was washed with 10 mL of buffer A, then 3 mL of 0.1 M glycine-HCl (pH 2.8) was flowed to eliminate the fluorescent premix particles, thereby a fluorescent stain (No. 5) was obtained.

A stained slide was produced in the same manner as in Example 1 except that the obtained fluorescent stain (No. 5) was used, and the fluorescently-stained image was photographed. In the obtained fluorescently-stained image, bright spots derived from the aggregated fluorescent premix particles were hardly found, and the average brightness was maintained at a high value of 540.

### [Example 6] Preparation of fluorescent stain (No. 6) containing primary aptamer-linked Texas Red integrated silica particles and photographing of fluorescently-stained image using the same

The streptavidin-modified Texas Red integrated silica particle dispersion liquid at a concentration of 0.02 nM obtained in Production Example 4 (25 µL) and the biotin-modified HER2 antigen-recognizing RNA aptamer (a primary aptamer) solution at a concentration of 6 µg/mL obtained in Production Example 10 (25 µL) were mixed and reacted at room temperature for 1 hour to prepare a fluorescent stain (No. 6) containing HER2 antigen-recognizing RNA aptamer (a primary aptamer) -linked Texas Red integrated silica particles (fluorescent premix particles).

A stained slide was produced in the same manner as in Example 1 except that the obtained fluorescent stain (No. 6) was used, and the fluorescently-stained image was photographed. The average brightness of the obtained fluorescently-stained image was 1701.

### [Example 7] Preparation of fluorescent stain (No. 7) containing secondary aptamer-linked Texas Red integrated melamine resin particles and photographing of fluorescently-stained image using the same

The streptavidin-modified Texas Red integrated melamine resin particles dispersion liquid at a concentration of 0.02 nM obtained in Production Example 5 (25 µL) and
the biotin-modified rabbit IgG-recognizing RNA aptamer (a secondary aptamer) solution at a concentration of 6 µg/mL obtained in Production Example 11 (25 µL) were mixed and reacted at room temperature for 1 hour to prepare a fluorescent stain (No. 7) containing rabbit IgG-recognizing RNA aptamer (a secondary aptamer) -linked Texas Red integrated melamine resin particles (fluorescent premix particles).

In Comparative Example 1, a stained slide was prepared in the same manner as in Comparative Example 1 except that the method in (2-1A) above was changed to the method in (2-1C) below, and the fluorescently-stained image was photographed. The average brightness of the obtained fluorescently-stained image was 1920.

### (2-1C) Immunostaining

The activated (1-2) tissue array slide was immunostained according to the procedure of (i) and (ii) below.
(i) Primary reaction: a 0.05 nM solution of Anti-Erb2 antibody [EP1045Y] (manufactured by Abcam plc.), a rabbit monoclonal antibody, was dropped onto a tissue section on the tissue array slide using PBS containing 1% BSA, and those were reacted at 4°C overnight.
(ii) Secondary reaction: the tissue array slide after the primary reaction was washed with PBS. Then, a liquid obtained by adjusting the concentration of the fluorescent stain (No. 7) to 0.02 nM using 50 mM Tris solution was dropped onto the tissue section on the tissue array slide after the washing, and those were reacted at 4°C overnight.

### [Example 8] Preparation of fluorescent stain (No. 8) containing two types of fluorescent premix particles and photographing of fluorescently-stained image using the same

The anti-DNP antibody-modified Pyrromethene 556 integrated melamine resin particle dispersion liquid at a concentration of 0.02 nM obtained in Production Example 6 (10 µL) and the DNP-modified anti-mouse IgG antibody (a secondary antibody) solution at a concentration of 6 µg/mL obtained in Production Example 12 (90 µL) were mixed and reacted at room temperature for 1 hour to prepare a fluorescent stain (No. 8A) containing anti-mouse IgG antibody (a secondary antibody) -linked Pyrromethene 556 integrated melamine resin particles as first fluorescent premix particles.

The anti-FITC antibody-modified Texas Red integrated silica particle dispersion liquid at a concentration of 0.02 nM obtained in Production Example 7 (10 µL) and the FITC-modified anti-rabbit IgG antibody (a secondary antibody) solution at a concentration of 6 µg/mL obtained in Production Example 13 (90 µL) were mixed and reacted at room temperature for 1 hour to prepare a fluorescent stain (No. 8B) containing anti-rabbit IgG antibody (a secondary antibody) -linked Texas Red integrated silica particles as second fluorescent premix particles.

The obtained fluorescent stains (No. 8A) and (No. 8B) were mixed to prepare a one-liquid type fluorescent stain (No. 8) for double staining containing equal amounts of the first and second fluorescent premix particles.

Lung tissue array slide "LC241b" manufactured by US Biomax Inc. (a slide glass on which 25 sections in total of 2 sections each of tumor tissues and normal tissues from 6 patients and 1 section of pheochromocytoma (a tissue marker) are placed) was purchased. Using the tissue array slide, a specimen pretreatment step (deparaffinization, activation), a staining step (doubleimmunostaining, fixation of phosphor integrated particles, and staining for morphology observation), and a specimen post-treatment step (sealing) were performed according to the procedure shown in (1), (2), and (3) below to produce a stained slide based on immunostaining method. Then, using the produced stained slide, observation and imaging were performed according to the procedure shown in (4) below.

### (1) Specimen pretreatment step

### (1-1) Deparaffinization

(i) The tissue array slide was immersed in a container containing xylene for 15 minutes. During immersion, xylene was replaced twice.
(ii) The tissue array slide obtained in (i) was immersed in a container containing ethanol for 10 minutes. During immersion, ethanol was replaced twice.

### (1-2) Activation

(i) The deparaffinized tissue array slide was immersed in a container containing water for 10 minutes.
(ii) The tissue array slide obtained in (i) was immersed in 10 mM citrate buffer (pH 6.0).
(iii) The slide was autoclaved at 121°C for 5 minutes.
(iv) The tissue array slide after the autoclaving was immersed in a container containing PBS for 15 minutes. During immersion, PBS was replaced 3 times.
(v) The tissue array slide obtained in (iv) was brought into contact with PBS containing 1% BSA and allowed to stand for 1 hour.

### (2) Staining step

### (2-1) Doubleimmunostaining

(i) A solution containing anti-PD-Ll mouse antibody and anti-EGFR rabbit antibody each diluted to a concentration of 0.05 nM with PBS containing 1% BSA was placed on the activated tissue array slide, and those were allowed to stand overnight.
(ii) The one-liquid type fluorescent stain (No. 8) was diluted with PBS containing 1% BSA so that the concentration of each type of premix particles would be 0.1 nM, the diluted stain was placed onto the tissue array slide obtained in (i), and those were allowed to stand overnight.

### (2-2) Fixation of phosphor integrated particles and staining for morphology observation

(i) The tissue array slide after the doubleimmunostaining was immersed in a container containing PBS for 30 minutes.
(ii) The tissue array slide obtained in (i) was immersed in a solution of 4% neutral paraformaldehyde for 10 minutes to fix the phosphor integrated particles, then HE staining was performed to produce a stained slide.

### (3) Specimen post-treatment step

Aquatex, manufactured by Merck & Co., was dropped onto the obtained stained slide, and then a cover glass was placed thereon for sealing.

### (4) Observation

The stained slide after the sealing was placed on the stage of a fluorescence microscope (manufactured by Carl Zeiss) equipped with two types of filter sets (manufactured by Semrock Inc, see Table 3 below) for green and red. In each of both filter sets, the total brightness value calculated from the fluorescent bright spots in the entire screen of the fluorescent image of the stained slide and the brightness value for each bright spot were measured. The brightness ratio between the two types of fluorescent premix particles was calculated according to the "first calculation method" and the "second calculation method" described in WO 2017/014196 and found to be the same (see Table 4), and it was confirmed that PD-L1 and EGFR were expressed at a ratio of PD-L1 : EGFR = 100 : 1900 (Table 5).

**[Table 3]**

| Filter set | Excitation wavelength/emission wavelength of filter set | |
|---|---|---|
| | For green | For red |
| | (for Pyrromethene 556) | (for Texas Red) |
| Excitation filter | 470 nm | 586 nm |
| | (range of wavelength: 30 nm) | (range of wavelength: 20 nm) |
| Beam splitter | 495 nm | 605 nm |
| Fluorescent filter | 525 nm | 628 nm |
| | (range of wavelength: 50 nm) | (range of wavelength: 32 nm) |

**[Table 4]**

| Source of fluorescent bright spot | Brightness ratio | |
|---|---|---|
| | Filter set for green | Filter set for red |
| First premix particles (green): anti-mouse IgG antibody-linked Pyrromethene 556 integrated melamine resin particles | 1 | 0.08 |
| Second fluorescent premix particles (red): anti-rabbit IgG antibody-linked Texas Red integrated silica particles | 0.12 | 1 |

**[Table 5]**

| Source of fluorescent bright spot | Number of fluorescent bright spots | |
|---|---|---|
| | Filter set for green (for Pyrromethene 556) | Filter set for red (for Texas Red) |
| Stained slide | 1412 | 312 |
| First premix particles: anti-mouse IgG antibody-linked Pyrromethene 556 integrated melamine resin particles | L (1) = 1900 | 0.08L (1) = 142 |
| Second fluorescent premix particles: anti-rabbit IgG antibody-linked Texas Red integrated silica particles | 0.12L (2) = 14 | L (2) = 100 |

### Reference Signs List

1 Fluorescent premix particle (target protein-binding substance-linked phosphor integrated particle)
10 Phosphor integrated particle
15 Linker
20 First reactive substance
21 Streptavidin
30 Second reactive substance
31 Biotin
40 Target protein-binding substance
41 Primary antibody
42 Secondary antibody
100 Tissue section
150 Target protein

## Claims

1. Fluorescent premix particles, comprising: phosphor integrated particles that are modified with a first reactive substance; and at least one target protein-binding substance that is modified with a second reactive substance and is selected from the group consisting of an antibody and an aptamer, wherein the phosphor integrated particles and the at least one target protein-binding substance are linked by interaction between the first reactive substance and the second reactive substance.

2. The fluorescent premix particles according to claim 1, wherein the first reactive substance is streptavidin and the second reactive substance is biotin.

3. The fluorescent premix particles according to claim 1 or 2, wherein the target protein-binding substance is a primary antibody or a primary aptamer that specifically binds to a target protein.

4. The fluorescent premix particles according to claim 1 or 2, wherein the target protein-binding substance is a secondary antibody or a secondary aptamer that specifically binds to a primary antibody that specifically binds to a target protein.

5. The fluorescent premix particles according to any one of claims 1 to 4, wherein the phosphor integrated particles are particles including a resin base and a phosphor.

6. The fluorescent premix particles according to any one of claims 1 to 5, wherein the phosphor integrated particles have a number of the target protein-binding substances linked per unit area of a surface of the phosphor integrated particles of 0.003 to 0.012 target protein-binding substances/nm².

7. A fluorescent stain containing the fluorescent premix particles according to any one of claims 1 to 6.

8. The fluorescent stain according to claim 7, wherein the fluorescent stain contains two or more types of the fluorescent premix particles having peaks of emission wavelength that are separated from each other by 100 nm or more and is a one-liquid type or a multi-liquid type.

9. A fluorescent staining method in which a fluorescent stain containing the fluorescent premix particles according to claim 3 is used, the method comprising:
binding the fluorescent premix particles in the fluorescent stain to a target protein.

10. A fluorescent staining method in which a fluorescent stain containing the fluorescent premix particles according to claim 4 is used, the method comprising:
binding the primary antibody to a target protein; and
binding the fluorescent premix particles in the fluorescent stain to the primary antibody bound to the target protein.

11. A fluorescent staining method in which the fluorescent stain according to claim 8 is used, the method comprising:
directly or indirectly binding each of the fluorescent premix particles in the fluorescent stain that corresponds to each of two or more types of target proteins to the each of two or more types of target proteins.


SEQUENCE LISTING

<110>  Konica Minolta,Inc.

<120> Fluorescent premix particles, fluorescent stain containing same,
and fluorescent staining method in which these are used

<130>   B41545WO01

<160>  2

<170>  PatentIn version 3.5

<210>  1
<211>  34
<212>  RNA
<213>  Artificial sequence

<220>
<223>  biotinylated HER2antigen recognizing aptamer

<400>  1
agccgcgagg ggagggauag gguagggcgc ggcu                                34


<210>  2
<211>  73
<212>  RNA
<213>  Artificial sequence

<220>
<223>  Biotinylated rabbit IgG recognizing aptamer

<400>  2
gggagaauuc cgaccagaag uucgauacgc cgugggguga cguuggcuac ccuuuccucu      60

cuccuccuuc uuc                                                       73